# EUROPEAN PATENT SPECIFICATION

(11) **EP 3 991 681 B1**
(45) Date of publication and mention of the grant of the patent: **11.11.2026**
(21) Application number: 21204799.7
(22) Date of filing: 26.10.2021
(51) Int. Cl.: A61B 18/14, A61B 5/287, A61B 5/00

(54) **SURFACE MOUNTED ELECTRODE CATHETER**
OBERFLÄCHENMONTIERTER ELEKTRODENKATHETER
CATHÉTER À ÉLECTRODE À MONTAGE EN SURFACE

(30) Priority: 27.10.2020 US 202017081918; 01.04.2021 CN 202110355698
(43) Date of publication of application: 04.05.2022
(73) Proprietor: Biosense Webster (Israel) Ltd, Yokneam, 2066717 (IL)
(72) Inventor: GOVARI, Assaf, 2066717 Yokneam (IL); BEECKLER, Christopher Thomas, Irvine, 92618 (US); KEYES, Joseph Thomas, Irvine, 92618 (US); HERRERA, Kevin Justin, Irvine, 92618 (US)
(74) Representative: Carpmaels & Ransford LLP

(56) References cited:
- WO-A1-2014/124231
- US-A1- 2012 271 136
- US-A1- 2019 282 116
- US-A1- 2022 071 695

## Description

### FIELD OF THE INVENTION

The present invention is defined by the appended claims and relates to medical devices, and in particular, catheters with electrodes and a method of manufacturing thereof.

### BACKGROUND

Throughout the application the unit degrees Centigrade is equivalent to degrees Celsius.

A wide range of medical procedures involve placing probes, such as catheters, within a patient's body. Location sensing systems have been developed for tracking such probes. Magnetic location sensing is one of the methods known in the art. In magnetic location sensing, magnetic field generators are typically placed at known locations external to the patient. A magnetic field sensor within the distal end of the probe generates electrical signals in response to these magnetic fields, which are processed to determine the coordinate locations of the distal end of the probe. These methods and systems are described in U.S. Pat. Nos. 5,391,199, 6,690,963, 6,484,118, 6,239,724, 6,618,612 and 6,332,089, in PCT International Publication No. WO 1996/005768, and in U.S. Patent Application Publications Nos. 2002/0065455 and 2003/0120150 and 2004/0068178. Locations may also be tracked using impedance or current based systems.

One medical procedure in which these types of probes or catheters have proved extremely useful is in the treatment of cardiac arrhythmias. Cardiac arrhythmias and atrial fibrillation in particular, persist as common and dangerous medical ailments, especially in the aging population.

Diagnosis and treatment of cardiac arrhythmias include mapping the electrical properties of heart tissue, especially the endocardium and the heart volume, and selectively ablating cardiac tissue by application of energy. Such ablation can cease or modify the propagation of unwanted electrical signals from one portion of the heart to another. The ablation process destroys the unwanted electrical pathways by formation of non-conducting lesions. Various energy delivery modalities have been disclosed for forming lesions, and include use of microwave, laser and more commonly, radiofrequency energies to create conduction blocks along the cardiac tissue wall. In a two-step procedure, mapping followed by ablation, electrical activity at points within the heart is typically sensed and measured by advancing a catheter containing one or more electrical sensors into the heart and acquiring data at a multiplicity of points. These data are then utilized to select the endocardial target areas at which the ablation is to be performed.

Electrode catheters have been in common use in medical practice for many years. They are used to stimulate and map electrical activity in the heart and to ablate sites of aberrant electrical activity. In use, the electrode catheter is inserted into a major vein or artery, e.g., femoral vein, and then guided into the chamber of the heart of concern. A typical ablation procedure involves the insertion of a catheter having a one or more electrodes at its distal end into a heart chamber. A reference electrode may be provided, generally taped to the skin of the patient or by means of a second catheter that is positioned in or near the heart. RF (radio frequency) current is applied between the tip electrode(s) of the ablating catheter, and the reference electrode, flowing through the media between the electrodes it, i.e., blood and tissue. The distribution of current depends on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive.

US Patent Publication 2014/0276733 of VanScoy, et al., describes an ablation catheter including an elongated body having a proximal end and a distal end. At least one ablation element is disposed on the body between the proximal end and the distal end and configured to ablate renal tissue to control hypertension. At least one localization sensor is disposed on the body and configured to interact with a magnetic field. The at least one localization sensor aids in determining an appropriate target tissue for ablation.

US Patent Publication 2008/0125772 of Stone, et al., describes a catheter and catheter system using energy tailored for remodeling and/or removal of target material along a body lumen, often of atherosclerotic material of a blood vessel of a patient. An elongate flexible catheter body with a radially expandable structure may have a plurality of electrodes or other electrosurgical energy delivery surfaces to radially engage atherosclerotic material when the structure expands. An atherosclerotic material detector system may measure and/or characterize the atherosclerotic material and its location, optionally using impedance monitoring.

US Patent Publication 2011/0137298 of Nguyen, et al., describes an ablation apparatus comprising an ultrasonic transducer which includes a piezoelectric element having a cylindrical shape; a plurality of external electrodes disposed on the outer surface of the piezoelectric element; and at least one internal electrode disposed on the inner surface of the piezoelectric element. The internal electrode(s) provides corresponding internal electrode portions that are disposed opposite the external electrodes with respect to the piezoelectric element, the external electrodes and the at least one internal electrode to be energized to apply an electric field across the piezoelectric element. The ultrasonic ablation zones of the external electrodes are distributed in a staggered configuration so as to span one or more open arc segments around the longitudinal axis, and the ultrasound ablation zones of all external electrodes projected longitudinally onto any lateral plane which is perpendicular to the longitudinal axis span a substantially closed loop around the longitudinal axis.

US Patent Publication 2012/0067640 of Moulin, et al., describes an electrical connection making it possible to achieve leak-tight electrical linking in an environment that is subjected to corrosive gases or liquids, wherein the connection comprises: at least two conductors for forming said electrical connection between each other or with a connector element; an outer insulating layer formed on each of said conductors and made up of a fluorinated polymer that is meltable at a temperature situated between the temperature of said environment and a predetermined higher temperature; a heat-shrink sleeve surrounding said conductors and made up of an outer layer of heat-shrink polymer and an inner layer of said fluorinated polymer that is meltable at a temperature situated between the temperature of said environment and said predetermined higher temperature; melting said outer insulating layers of said conductors and said inner layer of said heat-shrink sleeve by heating to a temperature higher than said temperature of said environment and lower than said predetermined higher temperature, thereby making a weld that is continuous and leak-tight, and of controlled thickness.

US Patent Publication 2013/0131661 of Jackson, et al., describes a method and apparatus for treating abnormal mucosa in the esophagus, such that the depth of the treated tissue is controlled. The depth of ablation is controlled by monitoring the tissue impedance and/or the tissue temperature. A desired ablation depth is also achieved by controlling the energy density or power density, and the amount of time required for energy delivery. A method and apparatus are disclosed for measuring an inner diameter of a body lumen, where a balloon is inflated inside the body lumen at a fixed pressure.

US Patent 3,359,525 describes an electric heating element heat-stable up to temperatures of about 316 degrees Centigrade (600 degrees Fahrenheit), which comprises: (a) an electric heat generating unit comprising carbon dispersed in a binder of a material selected from the group consisting of aromatic polyimides, aromatic polyamides and aromatic polybenzimidazoles, said unit having means for connecting it to an electric power source; and (b) an electrically-insulating heat conductive coating in adherent contact with the surface of said unit, said coating comprising a polymer selected from the group consisting of aromatic polyimides, aromatic polyamides and aromatic polybenzimidazoles.

International Publication WO 2014/0124231 of Chou, et al., describes a flex-PCB catheter device that is configured to be inserted into a body lumen. The flex-PCB catheter comprises an elongate shaft, an expandable assembly, a flexible printed circuit board (flex-PCB) substrate, a plurality of electronic components and a plurality of communication paths. The elongate shaft comprises a proximal end and a distal end. The expandable assembly is configured to transition from a radially compact state to a radially expanded state. The plurality of electronic elements are coupled to the flex-PCB substrate and are configured to receive and/or transmit an electric signal. The plurality of communication paths are positioned on and/or within the flex-PCB substrate. The communication paths selectively couple the plurality of electronic elements to a plurality of electrical contacts configured to electrically connect to an electronic module configured to process the electrical signal. The flex-PCB substrate can have multiple layers, including one or more metallic layers. Acoustic matching elements and conductive traces can be includes in the flex-PCB substrate.

US Patent Publication 2019/0282116 of Olson describes flexible high-density mapping catheters with a high-density array of mapping electrodes. These mapping catheters may be used to detect electrophysiological characteristics of tissue in contact with the electrodes, and may be used to diagnose cardiac conditions, such as cardiac arrhythmias for example.

US Patent Publication 2012/0271136 of Kordis, et al., describes a method for sensing multiple local electric voltages from endocardial surface of a heart. The method includes: providing a system for sensing multiple local electric voltages from endocardial surface of a heart, including: a first elongate tubular member having a lumen, a proximal end and a distal end; a basket assembly including: a plurality of flexible splines for guiding a plurality of exposed electrodes, the splines having proximal portions, distal portions and medial portions therein between, wherein the electrodes are substantially flat electrodes and are substantially unidirectionally oriented towards a direction outside of the basket.

### SUMMARY OF THE INVENTION

The scope of the invention is defined by independent claims 1, 8 and 10. Further embodiments are defined in the dependent claims. Any methods for treatment of the human or animal body by surgery or therapy have been included solely to illustrate workings of the invention and are not claimed.

### SUMMARY OF THE DISCLOSURE

There is provided in accordance with an embodiment of the present disclosure, a medical system including a catheter configured to be inserted into a body part of a living subject, and including an elongated deflectable element including a distal end, a proximal coupler connected to the distal end, an expandable assembly including a plurality of flexible polymer circuit strips, the flexible polymer circuit strips having respective proximal ends connected to, and disposed circumferentially around, the proximal coupler, respective ones of the flexible polymer circuit strips including respective multiple strip electrodes, respective contact arrays disposed at the respective proximal ends, and respective multiple circuit traces electrically connecting the respective multiple strip electrodes with the respective contact arrays, and a plurality of surface mountable electrodes mounted on, and bulging over respective ones of the flexible polymer circuit strips.

Further in accordance with an embodiment of the present disclosure the catheter further includes first insulated electrical wires disposed in the elongated deflectable element, respective groups of the first insulated electrical wires being electrically connected to the respective contact arrays of respective ones of the flexible polymer circuit strips, and second insulated electrical wires disposed in the elongated deflectable element, respective ones of the second insulated electrical wires being run externally to respective ones of the flexible polymer circuit strips and being electrically connected to respective ones of the surface mountable electrodes.

Still further in accordance with an embodiment of the present disclosure the first insulated electrical wires include electrically insulating material which is configured to have a temperature rating between 150 and 200 degrees Centigrade, and the second insulated electrical wires include electrically insulating material which is configured to have a temperature rating greater than 200 degrees Centigrade.

Additionally, in accordance with an embodiment of the present disclosure respective ones of the first insulated electrical wires have a first wire gauge, respective ones of the second insulated electrical wires have a second wire gauge, and the first wire gauge is greater than the second wire gauge.

Moreover, in accordance with an embodiment of the present disclosure the catheter includes respective shrink-sleeves securing respective ones of the second wires to respective ones of the flexible polymer circuit strips.

Further in accordance with an embodiment of the present disclosure respective ones of the surface mountable electrodes are mounted externally to the respective shrink-sleeves of respective ones of the flexible polymer circuit strips.

Still further in accordance with an embodiment of the present disclosure the catheter includes respective cable jackets disposed in the elongated deflectable element, the respective groups of the first insulated electrical wires are disposed in respective ones of the cable jackets, and respective ones of the second insulated electrical wires are disposed in respective ones of the cable jackets among the respective groups of the first insulated wires.

Additionally, in accordance with an embodiment of the present disclosure each respective one of the surface mountable electrodes extends around the respective flexible polymer circuit strip.

Moreover in accordance with an embodiment of the present disclosure, the system includes an ablation power generator configured to be connected to the catheter, and apply an electrical signal to at least one of the surface mountable electrodes to ablate a tissue of the body part, and a mapping module configured to receive electrical signals from ones of the strip electrodes of the flexible polymer circuit strips, and generate an electro-anatomical map responsively to the received electrical signals.

Further in accordance with an embodiment of the present disclosure the catheter includes a pusher including a distal portion, and being configured to be advanced and retracted through the deflectable element, the catheter includes a distal coupler connected to the distal portion of the pusher, and the flexible polymer circuit strips are disposed circumferentially around the distal portion of the pusher, the flexible polymer circuit strips have respective distal ends connected to the distal coupler, and the strips being configured to bow radially outward when the pusher is retracted expanding the expandable assembly from a collapsed form to an expanded form.

There is also provided in accordance with another embodiment of the present disclosure, a catheter device configured to be inserted into a body part of a living subject, and including an elongated deflectable element including a distal end, a distal end assembly disposed at the distal end and including a plurality of first electrodes and a plurality of second electrodes, first insulated electrical wires disposed in the elongated deflectable element, respective ones of the first insulated electrical wires being electrically connected to respective ones of the first electrodes, the first insulated electrical wires including electrically insulating material which is configured to have a temperature rating between 150 and 200 degrees Centigrade, and second insulated electrical wires disposed in the elongated deflectable element, respective ones of the second insulated electrical wires being electrically connected to respective ones of the second electrodes, the second insulated electrical wires including electrically insulating material which is configured to have a temperature rating greater than 200 degrees Centigrade.

Still further in accordance with an embodiment of the present disclosure, the device includes shrink-sleeves covering at least part of the second insulated electrical wires.

There is also provided in accordance with still another embodiment of the present disclosure, a method of manufacturing a catheter including providing a catheter including an elongated deflectable element, a proximal coupler connected to a distal end of the elongated deflectable element, and an expandable assembly including a plurality of flexible polymer circuit strips, the flexible polymer circuit strips having respective proximal ends connected to, and disposed circumferentially around, the proximal coupler, respective ones of the flexible polymer circuit strips including respective multiple strip electrodes, respective contact arrays disposed at the respective proximal ends, and respective multiple circuit traces electrically connecting the respective multiple strip electrodes with the respective contact arrays, and mounting a plurality of surface mountable electrodes over respective ones of the flexible polymer circuit strips with the surface mountable electrodes bulging over the respective ones of the flexible polymer circuit strips.

Additionally in accordance with an embodiment of the present disclosure, the method includes disposing first insulated electrical wires in the elongated deflectable element, electrically connecting respective groups of the first insulated electrical wires to the respective contact arrays of respective ones of the flexible polymer circuit strips, disposing second insulated electrical wires in the elongated deflectable element, running respective ones of the second insulated electrical wires externally to respective one of the flexible polymer circuit strips, and electrically connecting respective ones of the second insulated electrical wires to respective ones of the surface mountable electrodes.

Moreover, in accordance with an embodiment of the present disclosure the electrically connecting respective groups of the first insulated electrical wires includes melting electrically insulating material of the first wires, and the electrically connecting respective ones of the second insulated electrical wires includes mechanically stripping electrically insulating material of the second insulated electrical wires.

Further in accordance with an embodiment of the present disclosure respective ones of the first insulated electrical wires have a first wire gauge, respective ones of the second insulated electrical wires have a second wire gauge, and the first wire gauge is greater than the second wire gauge.

Still further in accordance with an embodiment of the present disclosure, the method includes shrink-wrapping respective ones of the second insulated electrical wires to respective ones of the flexible polymer circuit strips.

Additionally in accordance with an embodiment of the present disclosure, the method includes disposing respective shrink-sleeves around respective ones of the second insulated electrical wires and respective ones of the flexible polymer circuit strips, pulling respective ones of the second insulated electrical wires through respective holes in respective ones of the shrink-sleeves, heat shrinking respective distal portions of respective ones of the shrink-sleeves, sliding respective ones of the surface mountable electrodes over the respective heat shrunk distal portions of respective ones of the shrink-sleeves, electrically connecting respective ones of the second insulated electrical wires to respective ones of the surface mountable electrodes, removing slack in respective ones of the second insulated electrical wires, and heat shrinking respective proximal portions of respective ones of the shrink-sleeves.

Moreover, in accordance with an embodiment of the present disclosure, the method includes disposing the respective groups of the first insulated electrical wires in respective cable jackets, and disposing respective ones of the second insulated electrical wires in respective ones of the cable jackets among the respective groups of the first insulated wires.

### BRIEF DESCRIPTION OF THE DRAWINGS

The present invention will be understood from the following detailed description, taken in conjunction with the drawings in which:
Fig. 1 is a partly pictorial, partly block diagram view of a medical system constructed and operative in accordance with an embodiment of the present invention;
Fig. 2 is a schematic view of a catheter of the system of Fig. 1;
Fig. 3 is a partially exploded view of the catheter of Fig. 2;
Fig. 4A is schematic view of a distal end of the catheter of Fig. 2 without a distal electrode;
Figs. 4B-C are schematic views of a distal end of a flexible polymer circuit strip of the catheter of Fig. 2;
Fig. 5 is a cross-sectional view through line A:A of Fig. 1;
Fig. 6 is a cross-sectional view through line B:B of Fig. 1;
Figs. 7A-B are schematic views illustrating the catheter of Fig. 1 is a deployed form and a collapsed form, respectively;
Fig. 8 is a schematic view of a flexible polymer circuit strip of the catheter of Fig. 2;
Figs. 9 and 10 are schematic view of a flexible polymer circuit strip of the catheter of Fig. 2 showing circuit traces;
Fig. 11 is a schematic view of the flexible polymer circuit strip of Fig. 8 with a surface mountable electrode mounted thereon;
Fig. 12 is a more detailed view of the surface mountable electrode of Fig. 11;
Fig. 13 is a cross-sectional view through line A:A of Fig. 12;
Fig. 14 is a cross-sectional view through line B:B of Fig. 12;
Fig. 15 is a flowchart including steps in a method of manufacturing the catheter of Fig. 2;
Fig. 16 is an axial cross-sectional view of an elongated deflectable element of the catheter of Fig. 2;
Fig. 17 is an axial cross-sectional view of cable disposed in the elongated deflectable element of Fig. 16;
Fig. 18 is a schematic longitudinal cross-sectional view of an alternative flexible polymer circuit strip for use with the catheter of Fig. 2;
Fig. 19 is a flowchart including steps in a method of manufacture of a catheter including the flexible polymer circuit strip of Fig. 18; and
Fig. 20 is a flowchart including more detailed steps in a method of manufacture of a catheter including the flexible polymer circuit strip of Fig. 18.

### DESCRIPTION OF EXAMPLE EMBODIMENTS

### OVERVIEW

Diagnostic catheters, such as basket catheters, provide many electrodes to capture electrical signals, such as electrical potentials, from tissue of a body part of a patient. The electrical signals may then be analyzed to provide an indication of the medical state of the body part. For example, the electrical signals may be used to provide an electro-anatomical map. The medical state of the body part may indicate that some of the tissue may require ablation (such as radiofrequency (RF) ablation or irreversible electroporation (IRE)) using a suitable catheter. Although the basket catheter electrodes may be suitable for diagnostic purposes, the electrodes and/or the connections to the electrodes may be too small to be used for ablation. This is particularly true for IRE where high currents are used, and a large electrode surface area is needed to provide effective IRE ablation. Therefore, in such situations, the diagnostic basket catheter is removed from the patient, and a suitable ablation catheter is inserted into the body part of the patient, and ablation is performed. Using two catheters increases the length of the medical procedure, may delay essential therapeutic treatment, and may increase trauma of the patient due to the time delay and the use of two catheters.

Embodiments of the present invention solve the above problems by providing a basket catheter which includes both diagnostic electrodes and ablation electrodes thereby providing a combined therapeutic and diagnostic catheter.

The basket catheter includes a distal end expandable assembly made of flexible polymer circuit strips connected together proximally and distally to form a basket. Each strip includes diagnostic electrodes. Each strip also includes an ablation electrode connected to the strip. The ablation electrode of each strip may be slid over that strip, wrapped around that strip, or formed from two parts which are connected together around that strip.

In some embodiments, the ablation electrodes are connected to the flexible polymer circuit strips in a staggered formation with alternate ablation electrodes being disposed more proximally than other ablation electrodes to allow compact stowing of the expandable assembly during insertion of the catheter into the patient and removal of the catheter from the patient.

In some embodiments, each strip includes a contact pad, which is generally formed in the same or similar manner to the diagnostic electrodes. In disclosed embodiments, the contact pad is larger than the diagnostic electrodes. The ablation electrodes are connected to the respective contact pads using a suitable bonding method, such as using solder, conductive epoxy, resistance welding, laser welding, or any other suitable method, to provide an electrical connection between each ablation electrode and the respective contact pad. The ablation electrodes may be connected using one or two bonds. The ablation electrodes are also referred to herein as surface mountable electrodes. The diagnostic electrodes are also referred to herein as strip electrodes. Adhesive may also be used to connect the surface mountable electrodes to the strips in addition to the bonding.

In other embodiments, the ablation electrodes are electrically connected via wires run along the inner surface of the respective flexible polymer circuit strips and through an elongated deflectable element of the catheter to the proximal end of the catheter. Other wires run from the proximal end of the catheter through the deflectable element to respective contact arrays of the flexible polymer circuit strips thereby electrically connecting the diagnostic electrodes to the proximal end of the catheter.

In some embodiments, the wires from the ablation electrodes and the diagnostic electrodes are bundled in cables (e.g., in cable jackets) in the elongated deflectable element. In some embodiments, respective groups of the wires from the diagnostic electrodes are bundled with respective ones of the wires from the ablation electrodes thereby providing better insulation for the ablation electrode wires.

One problem encountered when manufacturing catheters is due to the miniaturization of the elements. For example, a basket catheter may include tens or even more than a hundred electrodes, each requiring a separate electrical connection from the basket to the proximal end of the catheter. Connecting the wires running from the proximal end of the catheter to the contact arrays (e.g., solder pads) of the flexible polymer circuit strips or other elements containing the electrodes is challenging. One solution is to use insulated wires with a low temperature rating (e.g., greater than 150 degrees Centigrade but less than 200 degrees Centigrade) so that the wire insulation melts or degrades when solder is applied to the wire ends (e.g., at about 300 degrees Centigrade) thereby allowing the wires to be connected to the contact arrays without having to strip the insulation from the wires. However, using low temperature rated wire may be problematic when dealing with high temperature situations during manufacture or use of the catheter. For example, heating thermal shrink-sleeves may melt or degrade (e.g., char and cause crumbling) the wire insulation and may lead to electrical shorts between the wires and conducting elements such as metal supports of the flexible polymer circuit strips.

Therefore, embodiments of the present invention solve the above problems by providing a catheter (e.g., a basket catheter or any other suitable catheter, such as, a balloon catheter, or a Lasso catheter) which uses insulated wires with a low temperature-rating (e.g., between 150 and 200 degrees Centigrade) to electrically connect electrodes where the wire insulation is not subject to melting or heat degradation other than when soldered to connections or where wire insulation is not important (e.g., the lack of insulation does not lead to shorts), and uses insulated wires with a higher temperature rating (e.g., more than 200 degrees Centigrade) to electrically connect electrodes where heat is applied the wires during manufacture and/or use of the catheter and could lead to shorts or other problems.

The term "temperature rating", as used in the specification and claims, is defined as the maximum continuous temperature that the wire insulation can withstand during its lifetime without causing thermal damage, such as melting or thermal degradation (e.g., charring and crumbling) of the wire insulation.

According to embodiments of the invention, the catheter uses wires with a low temperature rating (e.g., between 150 and 200 degrees Centigrade) (running from the proximal end of the catheter) which are electrically connected to the contact arrays (which in turn are electrically connected to diagnostic electrodes on the circuit strips via circuit traces) thereby allowing the wires to be connected using soldering without stripping the insulation from the wires. Other wires running from the proximal end of the catheter have a higher temperature rating (e.g., more than 200 degrees Centigrade)
which are stripped prior to being electrically connected to the ablation electrodes mounted on the circuit strips. The wires with the higher temperature rating may be connected to the respective circuit strips using thermal shrink-sleeves without be concerned that the application of heat to the shrink-sleeves will melt or degrade the insulation of the higher temperature rated wires.

In some embodiments, a mapping module run by a processor receives electrical signals from at least some of the strip electrodes of the flexible polymer circuit strips and generates an electro-anatomical map responsively to the received electrical signals. An ablation power generator is connected to the catheter and applies an electrical signal to one or more of the surface mountable electrodes to ablate (using RF or IRE ablation) the tissue of the body part. In some embodiments, the ablation power generator applies an electrical signal between at least some of the surface mountable electrodes.

In some embodiments, the catheter may include an electrode, referred to herein as a distal electrode, placed at the distal tip of the catheter between the distal ends of the strips. The distal electrode may be used for ablation. In some embodiments, the ablation power generator applies an electrical signal between one or more of the surface mountable electrodes and the distal electrode to ablate (using RF or IRE ablation) the tissue of the body part.

### SYSTEM DESCRIPTION

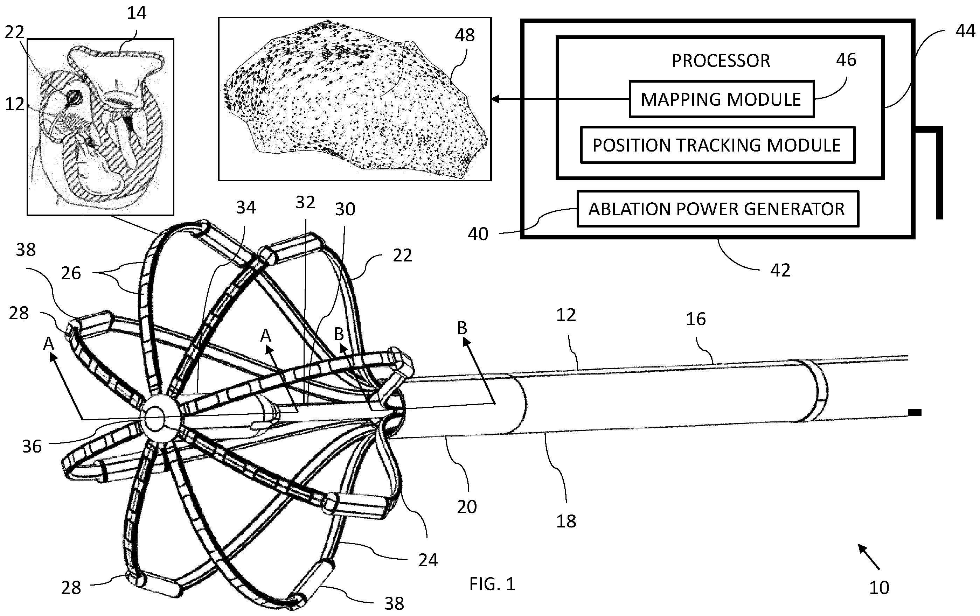

Reference is now made to Fig. 1, which is a partly pictorial, partly block diagram view of a medical system 10 constructed and operative in accordance with an embodiment of the present invention. The medical system 10 includes a catheter 12 configured to be inserted into a body part 14 (e.g., heart chamber) of a living subject.

The catheter 12 includes an elongated deflectable element 16 including a distal end 18. The elongated deflectable element 16 may have any suitable outer diameter and length, for example, the outer diameter may be in a range between 1 mm and 4 mm and the length may be in a range between 1 cm and 15 cm.

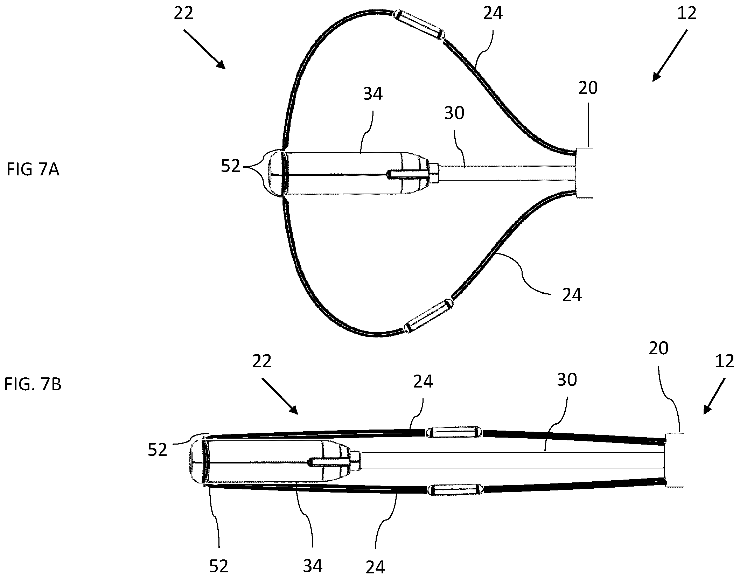

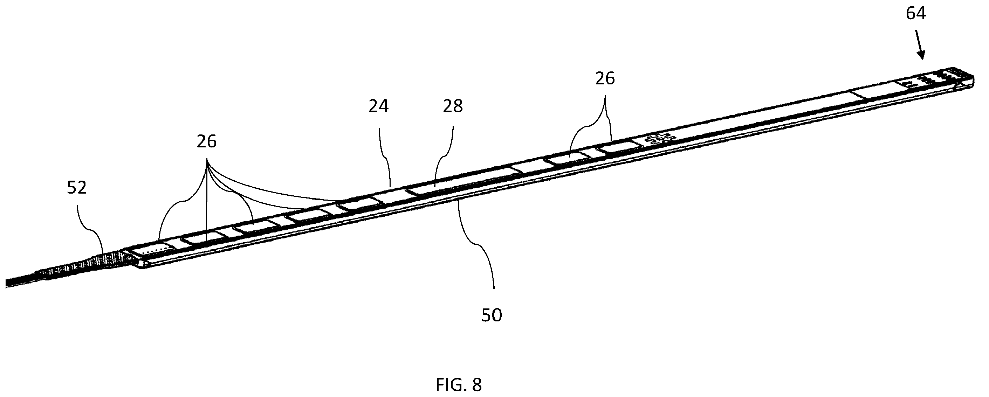

The catheter 12 also includes a proximal coupler 20 connected to the distal end 18. The proximal coupler 20 may be formed as an integral part of the deflectable element 16 or as a separate element, which is then connected to the distal end 18 using any suitable connection method, such as using adhesive, for example, epoxy. The catheter 12 also includes an expandable distal end assembly 22 including flexible polymer circuit strips 24 (only some labeled for the sake of simplicity). Each flexible polymer circuit strip 24 includes multiple strip electrodes 26 (only some labeled for the sake of simplicity) and a respective contact pad 28 disposed thereon. The contact pads 28 are mostly obscured in Fig. 1 and are shown more clearly in Figs. 8-10, 13, and 14. The catheter 12 includes a pusher 30 including a distal portion 32. The pusher 30 is configured to be advanced and retracted through the deflectable element 16. The catheter 12 also includes a distal coupler 34 connected to the distal portion 32 of the pusher 30. Proximal ends of the flexible polymer circuit strips 24 are connected to the proximal coupler 20, and distal ends of the flexible polymer circuit strips 24 are connected to the distal coupler 34 with the flexible polymer circuit strips 24 being disposed circumferentially around the distal portion 32 of the pusher 30. The flexible polymer circuit strips 24 are configured to bow radially outward when the pusher 30 is retracted expanding the expandable assembly from a collapsed form to an expanded form, described in more detail with reference to Figs. 7A-B.

In some embodiments, the catheter 12 includes a distal electrode 36 disposed at a distal tip of the catheter 12 between the distal ends of the flexible polymer circuit strips 24. In other embodiments, the catheter 12 includes a nose cap (not shown) disposed in the distal coupler 34 instead of the distal electrode 36.

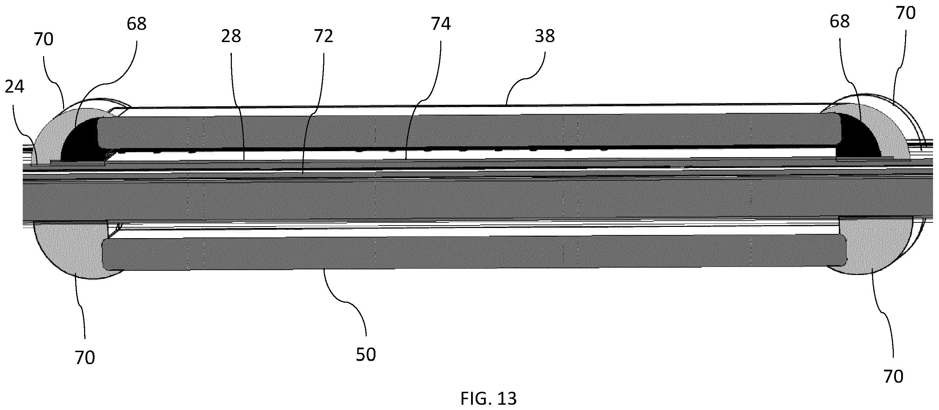

The catheter 12 includes surface mountable electrodes 38 electrically connected to respective one of the flexible polymer circuit strips 24. In some embodiments, one surface mountable electrode 38 is disposed on each flexible polymer circuit strip 24. The surface mountable electrodes 38 are electrically connected to the flexible polymer circuit strips 24 via the contact pads 28, as described in more detail with reference to Fig. 13.

The surface mountable electrodes 38 may be connected at the same position on each flexible polymer circuit strip 24 or in a staggered formation with alternate surface mountable electrodes 38 being disposed more proximally than other surface mountable electrodes 38 so that the expandable assembly 22 may compact efficiently when stowed during insertion into, and removal from the body part 14 of the patient.

In some embodiments, the surface mountable electrodes 38 may be connected to any suitable basket catheter with elements that differ from the elements of the catheter 12.

The medical system 10 includes an ablation power generator 40 configured to be connected to the catheter 12. The ablation power generator 40 may be housed in a console 42. The ablation power generator 40 is configured to apply an electrical signal to one or more of the surface mountable electrodes 38 to ablate tissue of the body part 14. In some embodiments, the ablation power generator 40 is configured to apply an electrical signal between some of the surface mountable electrodes 38 to ablate (using RF or IRE ablation) the tissue. In some embodiments, the ablation power generator 40 is configured to apply an electrical signal between one or more of the surface mountable electrodes 38 and the distal electrode 36 to ablate (using RF or IRE ablation) the tissue of the body part 14.

The medical system 10 includes a processor 44, which is configured to execute a mapping module 46 configured to: receive electrical signals from at least some of the strip electrodes 26 of the flexible polymer circuit strips 24; and generate an electro-anatomical map 48 responsively to the received electrical signals.

In practice, some or all of the functions of the processor 44 may be combined in a single physical component or, alternatively, implemented using multiple physical components. These physical components may comprise hardwired or programmable devices, or a combination of the two. In some embodiments, at least some of the functions of the processor 44 may be carried out by a programmable processor under the control of suitable software. This software may be downloaded to a device in electronic form, over a network, for example. Alternatively, or additionally, the software may be stored in tangible, non-transitory computer-readable storage media, such as optical, magnetic, or electronic memory.

The medical system 10 may include other modules and elements which are not shown for the sake of simplicity, such as an electrocardiogram module, a display screen, and user input devices (e.g., keyboard and mouse), by way of example only.

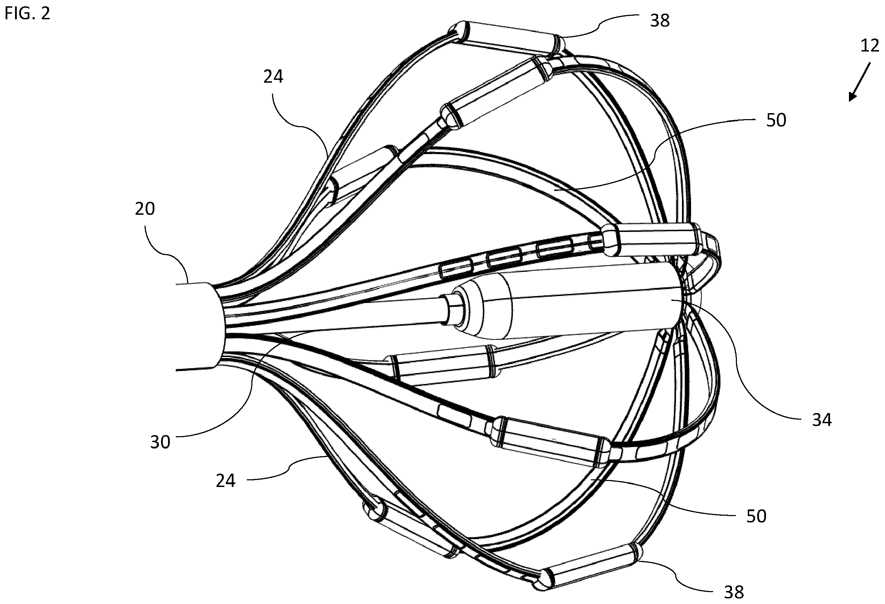

Reference is now made to Fig. 2, which is a schematic view of the catheter 12 of the system 10 of Fig. 1. Fig. 2 shows the proximal coupler 20, the distal coupler 34, the flexible polymer circuit strips 24 (only some labeled for the sake of simplicity), the pusher 30, and the surface mountable electrodes 38 (only some labeled for the sake of simplicity) in more detail.

The inner diameter of the pusher 30 is sized to accommodate wiring. The pusher 30 may be formed from any suitable material, for example, but not limited to polyimide with or without braiding, polyether ether ketone (PEEK) with or without braiding, or polyamide with or without braiding. The distal coupler 34 and the proximal coupler 20 may be formed from any suitable material, for example, but not limited to polycarbonate with or without glass filler, PEEK with or without glass filler, or PEI with or without glass filler.

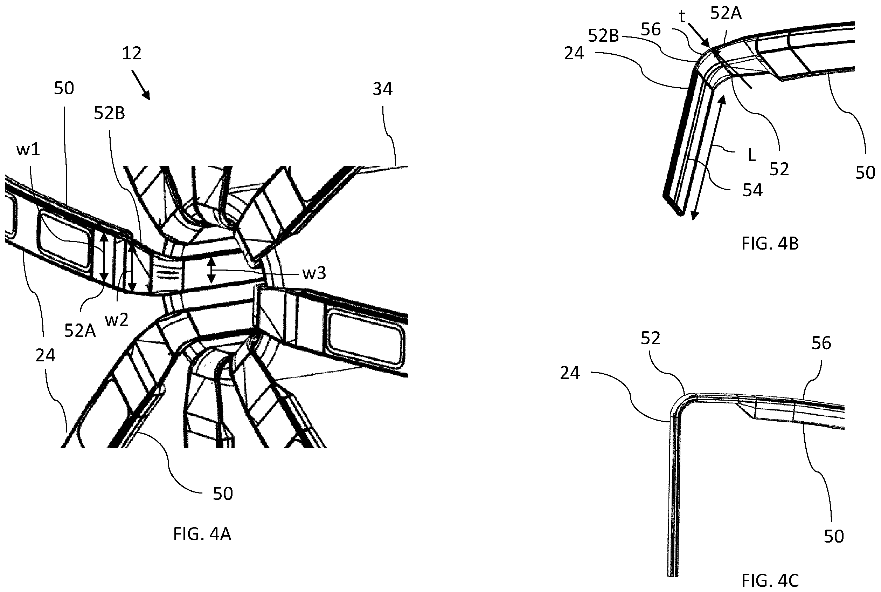

The catheter 12 includes elongated resilient support elements 50 (only some labeled for the sake of simplicity) connected along a given length of respective ones of the flexible polymer circuit strips 24 providing a shape of the expandable assembly 22 in the expanded form of the expandable assembly 22. The elongated resilient support elements 50 may include any suitable material, for example, but not limited to, Nitinol and/or Polyetherimide (PEI). The elongated resilient support elements 50 extend along the inner surface of the respective strips 24 from the proximal coupler 20 until just before the flexible polymer circuit strips 24 enter the distal coupler 34 to allow the flexible polymer circuit strips 24 to bend sufficiently at that point, as described in more detail with reference to Figs. 4A-C. The elongated resilient support elements 50 may have any suitable thickness, for example, in the range of about 0.025 mm to 0.25 mm.

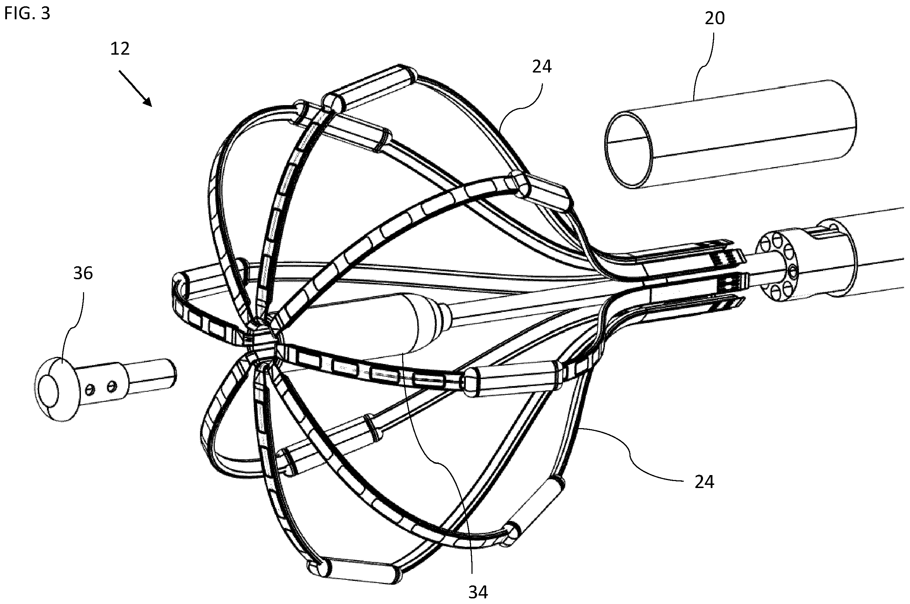

Reference is now made to Fig. 3, which is a partially exploded view of the catheter 12 of Fig. 2. Fig. 3 shows the catheter 12 with the distal electrode 36 (or nose piece) and the proximal coupler 20 removed to illustrate how the flexible polymer circuit strips 24 are connected to the distal coupler 34 and the proximal coupler 20. The proximal ends of the flexible polymer circuit strips 24 are connected to, and disposed circumferentially around, an inner surface of the proximal coupler 20. The distal ends of the flexible polymer circuit strips 24 are connected to, and disposed circumferentially around, an inner surface of the distal coupler 34. The flexible polymer circuit strips 24 may be connected to the proximal coupler 20 and distal coupler 34 using any suitable method, for example, using adhesive (e.g., epoxy) and/or using a pressure fit. The distal ends of the flexible polymer circuit strips 24 are generally bent over and connected to the distal end of the distal coupler 34 to allow the flexible polymer circuit strips 24 to bend at almost 90 degrees to form a flat nose catheter. **In** some embodiments, the distal ends of the flexible polymer circuit strips 24 may be connected to an outer surface of the distal coupler 34 or together without using a distal coupler.

Reference is now made to Fig. 4A, which is schematic view of a distal end of the catheter 12 of Fig. 2 without the distal electrode 36. Reference is also made to Figs. 4B-C, which are schematic views of a distal end of one of the flexible polymer circuit strips 24 of the catheter 12 of Fig. 2. Fig. 4A shows that the distal ends of the flexible polymer circuit strips 24 bend into the distal coupler 34. In some embodiments, the distal ends of the flexible polymer circuit strips 24 are tapered in order for the flexible polymer circuit strips 24 to fit into the distal coupler 34.

As previously mentioned, the flexible polymer circuit strips 24 are supported using the elongated resilient support elements 50 which extend from the proximal ends of the flexible polymer circuit strips 24 until a hinge section 52 of each flexible polymer circuit strip 24. The hinge section 52 may be reinforced using any suitable material. In some embodiments, the hinge section 52 is reinforced using a yarn 54 (Fig. 4B) that runs from between the flexible polymer circuit strip 24 and the elongated resilient support element 50 until the distal end of the flexible polymer circuit strip 24. The yarn 54 may comprises any one or more of the following: an ultra-high-molecular-weight polyethylene yarn; or a yarn spun from a liquid-crystal polymer. The yarn 54 may be any suitable linear density, for example, in a range between approximately 25 denier and 250 deniers. The flexible polymer circuit strip 24, elongated resilient support element 50, and yarn 54 may be connected together using any suitable method, for example, using an adhesive such as epoxy, and may be covered with a suitable covering 56 (Figs. 4B and 4C), e.g., thermoplastic polyethylene terephthalate (PET) shrink-sleeves. Windows are opened in the covering 56 to reveal the strip electrodes 26 and the contact pads 28 (Fig. 1).

It can also be seen in Fig. 4B that the hinge section 52 is much thinner, having a thickness "t", than the region including the elongated resilient support element 50. The hinge section 52 may have any suitable thickness, for example, in the range of about 10 to 140 microns. The hinge section 52 is provided with a first portion 52A having a width w1 (Fig. 4A) that tapers to a narrower width w2 in a second portion 52B. The hinge section 52, further narrows from width w2 (of 52B) to a width w3 in an end portion 52C. The width 52A is about 2 times width 52C. The length L of the final portion 52C is about 3 mm to ensure that the flexible polymer circuit strips 24 can be retained in coupler 34 without detaching.

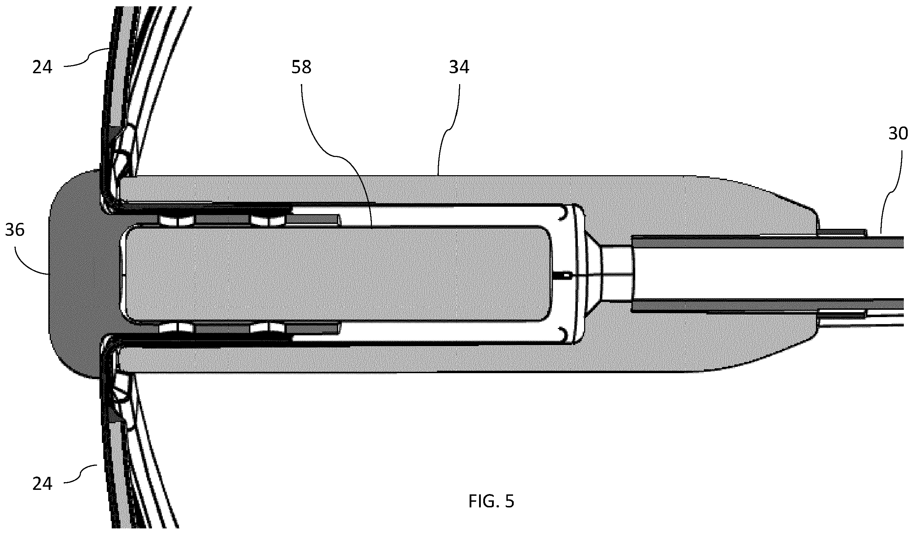

Reference is now made to Fig. 5, which is a cross-sectional view through line A:A of Fig. 1. Fig. 5 illustrates how the distal ends of the flexible polymer circuit strips 24 are connected to the inner surface of the distal coupler 34. A position sensor 58 (such as a magnetic position sensor) is optionally disposed in the distal coupler 34. The distal electrode 36 is inserted into the distal coupler 34 between the flexible polymer circuit strips 24 and the position sensor 58. Fig. 5 illustrates how the distal end of the pusher 30 is connected to the proximal end of the distal coupler 34.

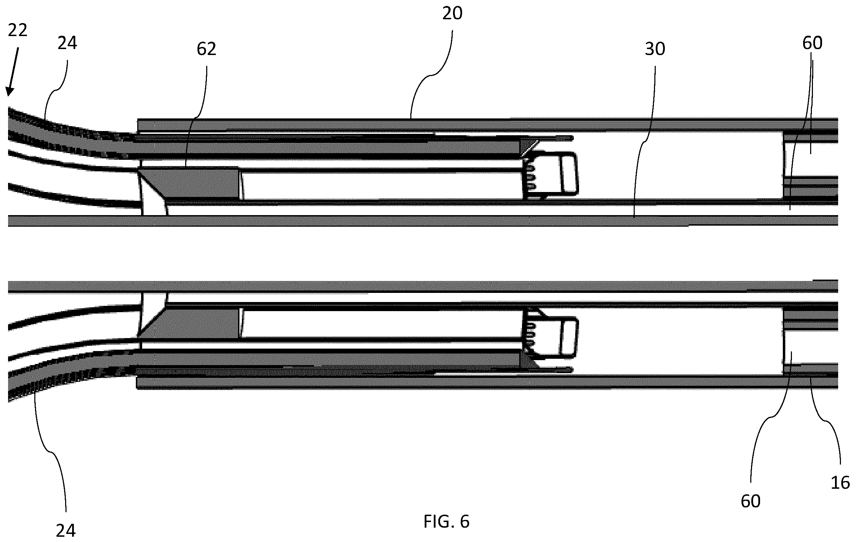

Reference is now made to Fig. 6, which is a cross-sectional view through line B:B of Fig. 1. Fig. 6 illustrates how the proximal ends of the flexible polymer circuit strips 24 are connected to the inner surface of the proximal coupler 20. Fig. 6 also shows that the proximal coupler 20 is connected around an outer surface of the deflectable element 16, which includes lumens 60 to carry wires, and irrigation tubes, for example, from the distal end to the proximal end of the catheter 12. The flexible polymer circuit strips 24 may be additionally held in place using a retaining ring 62. Fig. 6 also shows the pusher 30 extending from one of the lumens 60 of the deflectable element 16 into the expandable assembly 22.

Reference is now made to Figs. 7A-B, which are schematic views illustrating the catheter 12 of Fig. 1 is a deployed form and a collapsed form, respectively. The flexible polymer circuit strips 24 are configured to bow radially outward when the pusher 30 is retracted expanding the expandable assembly 22 from a collapsed form to an expanded form. The collapsed form of the expandable assembly 22 represents the non-stressed form of the flexible polymer circuit strips 24 which are provided with their shape using the elongated resilient support elements 50 (Figs. 4A-C). In some embodiments, the non-stressed form of the expandable assembly 22 is the expanded form. In some embodiments, the expandable assembly 22 collapses when retracted into a sheath (not shown) without needing a pusher or similar element.

In some embodiments, the flexible polymer circuit strips 24 are formed as flat strips. The distal ends of the flexible polymer circuit strips 24 are connected to the inner surface of the distal coupler 34 and then the proximal ends of the flexible polymer circuit strips 24 are connected to the proximal coupler 20 so that in the collapsed form, the angle between a tangent to the distal ends of the flexible polymer circuit strips 24 and an axis of the pusher 30 is close to 180 degrees, while in the expanded form, the angle between the tangent to the distal ends of the flexible polymer circuit strips 24 and the axis is about 90 degrees. Therefore, in operation (when the flexible polymer circuit strips 24 are connected to the distal electrode 36 and the proximal coupler 20) the hinge sections 52 are configured to provide a maximum angular range of movement of the flexible polymer circuit strips 24 of about 90 degrees and generally in excess of 80 degrees. However, the hinge section 52 is capable of bending 180 degrees or more.

Reference is now made to Fig. 8, which is a schematic view of one of the flexible polymer circuit strips 24 of the catheter 12 of Fig. 2. The flexible polymer circuit strip 24 shows the strip electrodes 26 and the contact pad 28. The contact pad 28 is typically formed in the same manner as the strip electrodes 26, except that the contact pad 28 may be longer than each strip electrode 26. **In** some embodiments, the contact pad 28 may be the same size as the strip electrodes 26. Fig. 8 also shows the hinge section 52 and the elongated resilient support element 50 on the lower side of the flexible polymer circuit strip 24. The flexible polymer circuit strip 24 also includes a contact array 64 for connecting the strip electrodes 26, and the contact pad 28 to wires which extend down the deflectable element 16 (Fig. 1) to the proximal end of the catheter 12.

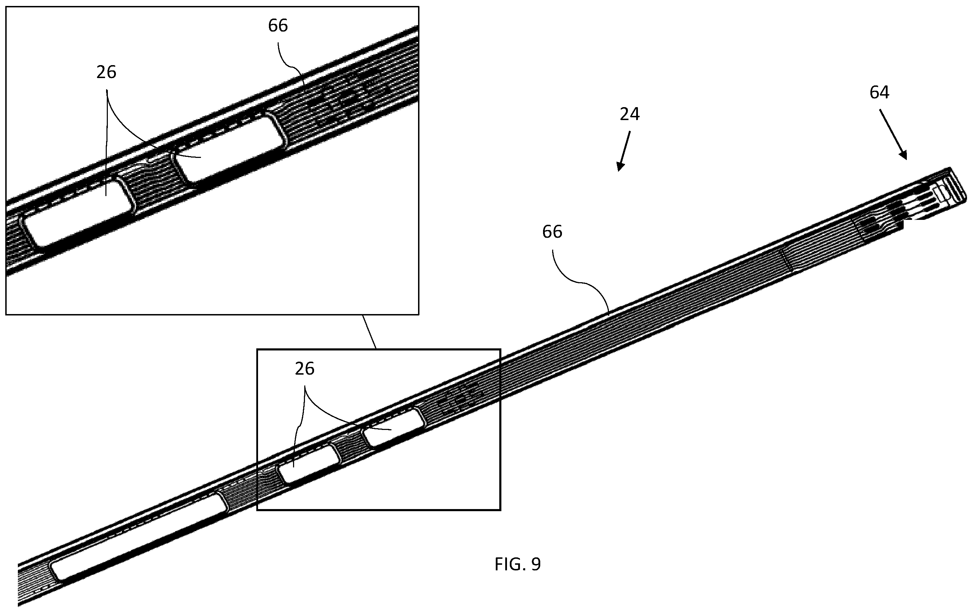

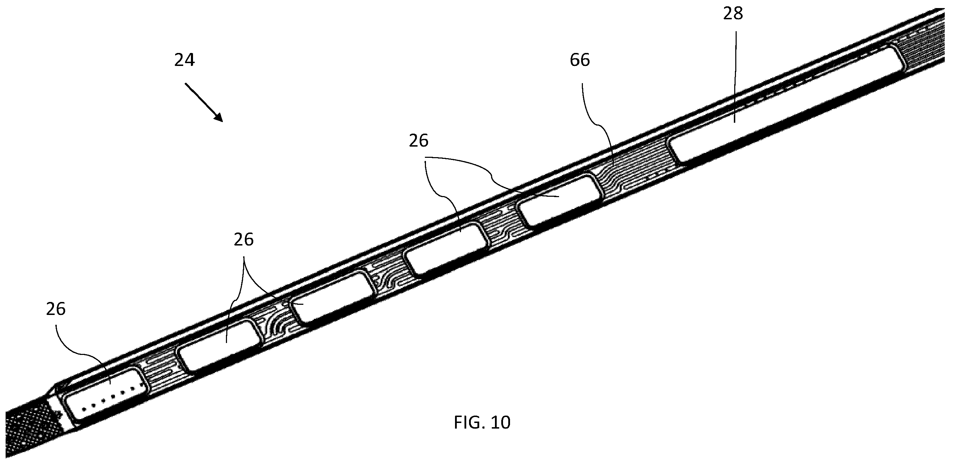

Reference is now made to Figs. 9 and 10, which are schematic view of one of the flexible polymer circuit strips 24 of the catheter 12 of Fig. 2 showing circuit traces 66. The flexible polymer circuit strip 24 of Figs. 9 and 10 are shown in a semi-transparent format to allow visualization of different layers of the flexible polymer circuit strip 24. The flexible polymer circuit strip 24 is generally formed from multiple layers including a lower and upper layer. A lower layer of the flexible polymer circuit strip 24 includes the circuit traces 66 which connect the strip electrodes 26 and the contact pad 28 to the contact array 64. The upper layer includes the strip electrodes 26 and the contact pad 28. The circuit traces 66 in the lower layer are connected to the strip electrodes 26 and the contact pad 28 in the upper layer using vias (not shown). The circuit trace 66 to the contact pad 28 may be wider than the other circuit traces 66 and may be spaced further apart from the other circuit traces 66 to ensure proper isolation. The circuit traces 66 for the strip electrodes 26 may be within the range of about 0.005 mm to 0.1 mm wide (e.g., 0.025 mm) with a spacing in the range of about 0.005 mm to 0.1 mm (e.g., 0.025 mm), while the trace 66 for the contact pad 28 may be in the range of about 0.025 mm to 0.25 mm wide (e.g., 0.125 mm wide) with a spacing to the nearest trace in the range of about 0.010 mm to 0.125 mm (e.g., 0.050 mm). The thickness of the traces 66 may be in the range of about 0.005 mm to 0.100 mm (e.g., 0.010 mm). In some embodiments, the circuit traces 66 may have the same width and spacing. The flexible polymer circuit strips 24 may be composed of any suitable materials. In some embodiments, each of the flexible polymer circuit strips 24 comprises a strip of polyimide. The circuit traces 66 are disposed on the back surface of the strip of polyimide and the strip electrodes 26 and contact pad 28 are disposed on the front surface of the strip of polyimide.

The flexible polymer circuit strips 24 may have any suitable dimensions. For example, the length of the flexible polymer circuit strips 24 may be in the range of 10 mm to 60 mm, e.g., 30 mm, the width of the flexible polymer circuit strips 24 may be in the range of about 0.25 mm to 3 mm, e.g., 0.72 mm, and the thickness of the flexible polymer circuit strips 24 may be in the range of about 0.005 mm to 0.14 mm.

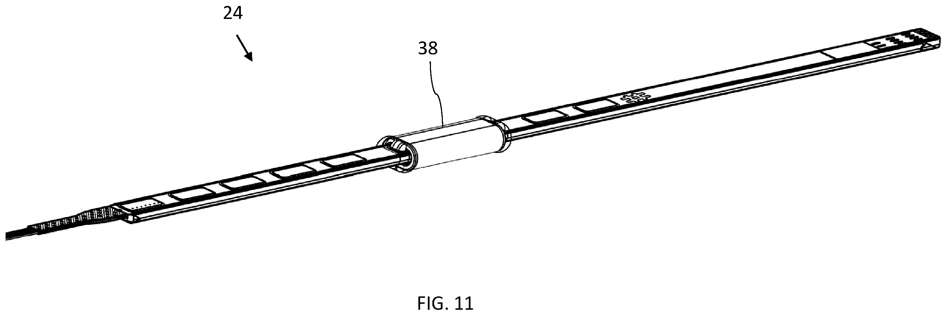

Reference is now made to Fig. 11, which is a schematic view of the flexible polymer circuit strip 24 of Fig. 8 having mapping electrodes 26 with one of the surface mountable electrodes 38 mounted thereon. The electrodes 26 are used to record electrocardiogram signals generated by the cardiac tissue. Each of the electrode 26 has a first exposed surface area A1. The surface mount electrode 38 can be used for ablation by delivering DC or AC signals via electrode 38. Electrode 38 has a second exposed surface area A2 of at least three times the first exposed area of each mapping electrode 26. The exposed surface area A1 is from about 0.08 mm-squared to about 1mm-squared. The surface mountable electrode 38 may be formed as a single element which is slid over the flexible polymer circuit strip 24 and the elongated resilient support element 50. In some embodiments, the surface mountable electrode 38 may be formed from two halves which are connected together around strip 24 and the elongated resilient support element 50. In yet other embodiments, the surface mountable electrode 38 may be formed as a band, which is wrapped around the flexible polymer circuit strip 24 and the elongated resilient support element 50. The surface mountable electrodes 38 may be formed from any suitable material, for example, but not limited to gold, gold alloys, platinum, platinum alloys, palladium, or palladium alloys.

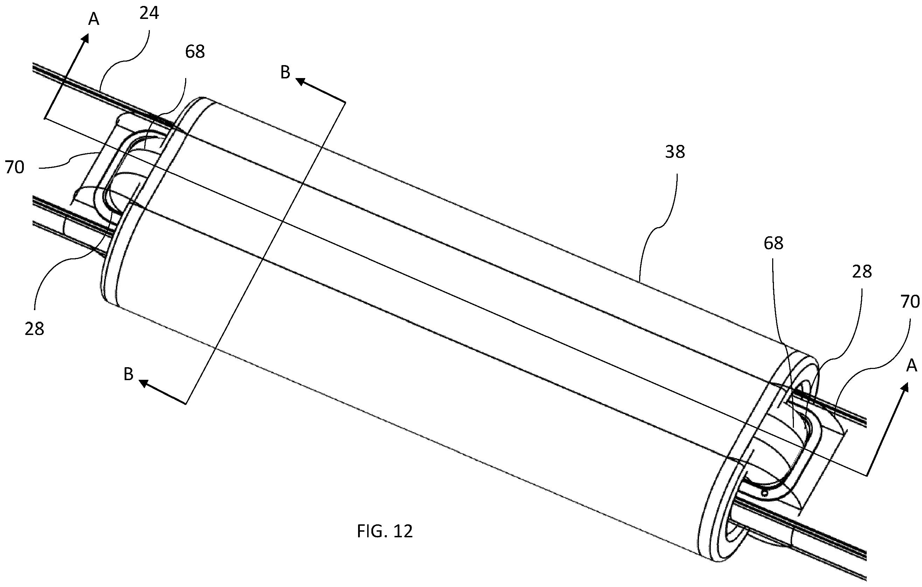

Reference is now made to Fig. 12, which is a more detailed view of the surface mountable electrode 38 of Fig. 11. The surface mountable electrode 38 is shown mounted over the contact pad 28. The surface mountable electrode 38 is electrically connected to the contact pad 28 using two electrically conductive retainers 68 (e.g., using solder, conductive epoxy, resistance welding, laser welding, or any other suitable method). The surface mountable electrode 38 is further secured to the flexible polymer circuit strip 24 using an adhesive 70, such as a polyurethane adhesive or epoxy. The adhesive 70 may be applied so that the contact pad 28 is electrically isolated from the environment around the catheter 12.

Reference is now made to Fig. 13, which is a cross-sectional view through line A:A of Fig. 12. Fig. 13 shows that the surface mountable electrode 38 extends over the contact pad 28 and below the elongated resilient support element 50. Fig. 13 also shows two layers of the flexible polymer circuit strip 24, a layer 72 including the circuit traces 66 (layer 72 shown in each of Figs. 9 and 10) and a layer 74 including the contact pad 28 and the strip electrodes 26 (layer 74 shown in Fig. 10). The contact pad 28 shown in Fig. 13 is wider than the surface mountable electrode 38. In some embodiments, the contact pad 28 may be narrower or the same width as the surface mountable electrode 38. The surface mountable electrode 38 is electrically connected to the contact pad 28 using at least one electrically conductive retainer 68. In some embodiments, the proximal end and the distal end of the surface mountable electrode 38 is electrically connected to the contact pad 28 using two respective electrically conductive retainers 68. The proximal end and the distal end of the surface mountable electrode 38 is optionally connected to the flexible polymer circuit strips 24 using adhesive 70. The adhesive 70 typically covers the remainder of the contact pad 28 that is not covered by the surface mountable electrode 38. The adhesive 70 may comprise polyurethane adhesive or epoxy, by way of example. While the bonds 68 are typically disposed on the outer surface of the flexible polymer circuit strip 24, the adhesive 70 is generally disposed around the flexible polymer circuit strip 24 and the elongated resilient support element 50 so as to secure the surface mountable electrode 38 to the flexible polymer circuit strip 24 and the elongated resilient support element 50 and prevent liquid contacting the contact pad 28 in use of the catheter 12.

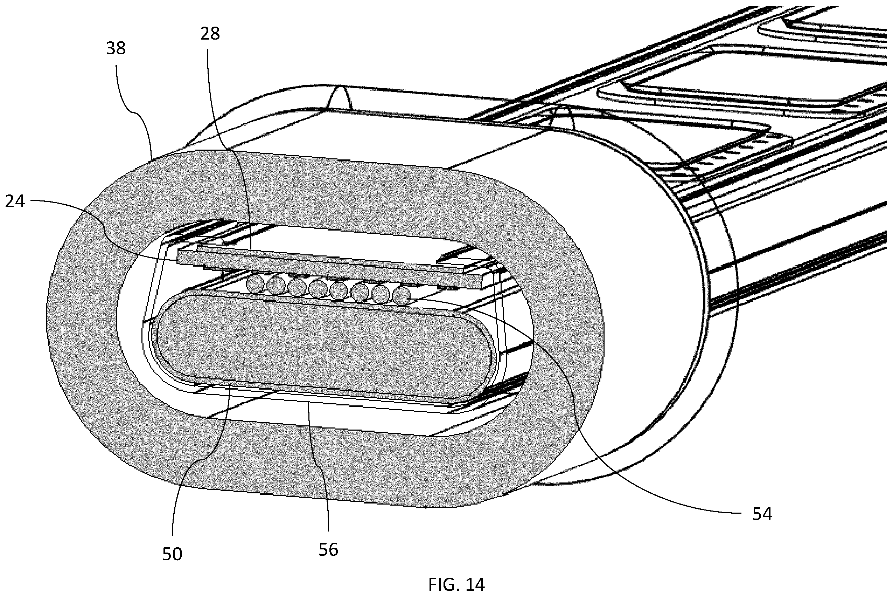

Reference is now made to Fig. 14, which is a cross-sectional view through line B:B of Fig. 12. Fig. 14 shows that the surface mountable electrode 38 extends around the flexible polymer circuit strip 24 and the elongated resilient support element 50. Fig. 14 also shows the yarn 54 sandwiched between the flexible polymer circuit strip 24 and the elongated resilient support element 50. The covering 56 (e.g., shrink-sleeves) is shown surrounding the elongated resilient support elements 50 and partially surrounding the flexible polymer circuit strips 24 where a window is opened in the covering 56 to expose the contact pad 28. The surface mountable electrode 38 shown in Fig. 14 is a one-piece hollowed electrode which is slid over the flexible polymer circuit strip 24 and elongated resilient support element 50 combination. As previously mentioned, the surface mountable electrode 38 may be formed from two halves which are connected together around strip 24. In yet other embodiments, the surface mountable electrode 38 may be formed as a band, which is wrapped around the flexible polymer circuit strip 24.

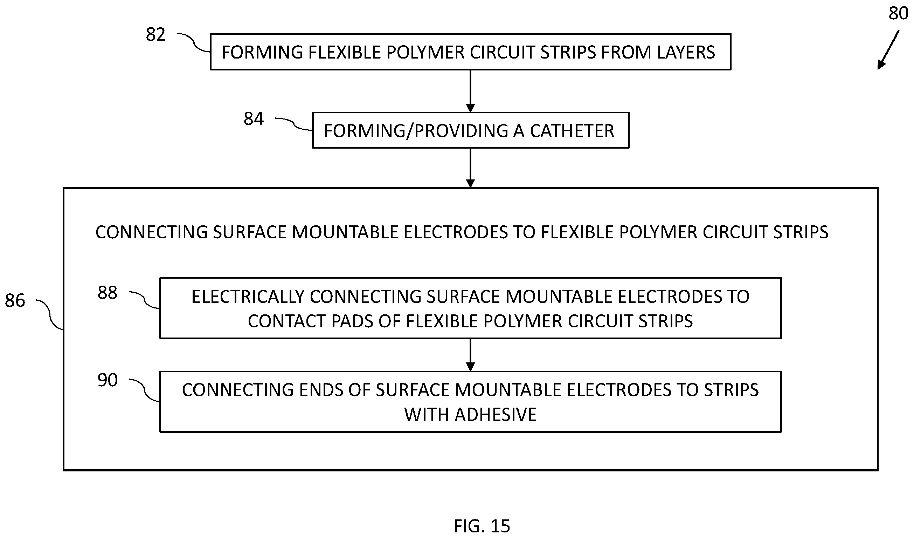

Reference is now made to Fig. 15, which is a flowchart 80 including steps in a method of manufacturing the catheter 12 of Fig. 2. Reference is also made to Fig. 1.

The method includes forming (block 82) each flexible polymer circuit strip 24 from multiple layers, with the layer 72 (Fig. 13) comprising circuit traces 66 (Figs. 9 and 10) and the layer 74 (Fig. 13) including the strip electrodes 26 and the respective contact pad 28.

The method includes forming or providing (block 84) the catheter 12 including: the elongated deflectable element 16; the proximal coupler 20 connected to the distal end 18 of the deflectable element 16; the distal coupler 34; the distal electrode 36; the pusher 30; and the expandable assembly 22. The expandable assembly 22 includes the flexible polymer circuit strips 24. Each flexible polymer circuit strip 24 includes the respective strip electrodes 26 and the respective contact pad 28 disposed thereon. The proximal ends and distal ends of the flexible polymer circuit strips 24 are connected to, and disposed circumferentially around, the proximal coupler 20 and the distal coupler 34, respectively. The distal electrode 36 is disposed at the distal tip of the catheter 12 between the distal ends of the flexible polymer circuit strips 24. The pusher 30 is configured to be advanced and retracted through the deflectable element 16. The distal portion of the pusher 30 is connected to the distal coupler 34. The flexible polymer circuit strips 24 are disposed circumferentially around the distal portion of the pusher 30. The flexible polymer circuit strips 24 are configured to bow radially outward when the pusher 30 is retracted expanding the expandable assembly 22 from a collapsed form to an expanded form.

The method also includes connecting (block 86) the surface mountable electrodes 38 to respective ones of the flexible polymer circuit strips 24. In some embodiments, the method includes connecting the surface mountable electrodes 38 to respective ones of the flexible polymer circuit strips 24 in a staggered formation with alternate ones of the surface mountable electrodes 38 being disposed more proximally that other ones of the surface mountable electrodes 38. The step of block 86 may include the sub-steps of blocks 88 and 90 as follows.

The method may also include electrically connecting (block 88) the surface mountable electrodes 38 to respective ones of the flexible polymer circuit strips 24 so that each surface mountable electrode 38 is electrically connected to the respective contact pad 28 of a respective one of the flexible polymer circuit strips 24 using at least one electrically conductive retainer 68 (Fig. 13), for example, using solder, conductive epoxy, resistance welding, laser welding, or any other suitable method. In some embodiments, the method includes electrically connecting the proximal and distal end of each surface mountable electrode 38 to the respective contact pad 28 of the respective flexible polymer circuit strip 24 using two respective electrically conductive retainers 68.

The method may also include connecting (block 90) the proximal and distal end of each surface mountable electrode 38 to the respective flexible polymer circuit strip 24 using adhesive 70.

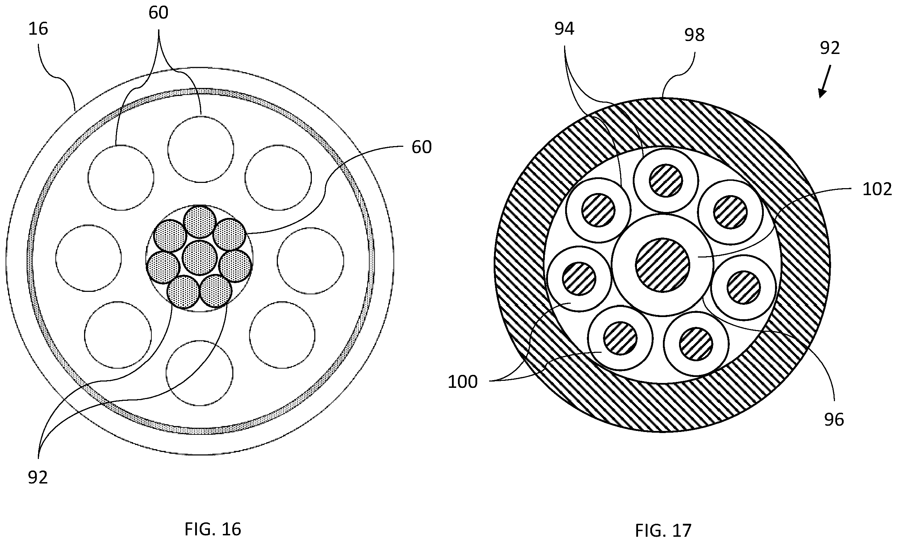

Reference is now made to Fig. 16, which is an axial cross-sectional view of the deflectable element 16 of the catheter 12 of Fig. 1. The deflectable element 16 includes lumens 60 (only some labeled for the sake of simplicity) disposed therein. The lumens 60 may be used to carry cables 92 (only some labeled for the sake of simplicity) therein, and/or other elements such as deflection wires, stiffening elements to create a deflection plane of the deflectable element 16, and a puller wire or the pusher 30 (Fig. 2). The cables 92 are shown disposed in the central lumen 60. The cables 92 may be disposed in one or more non-central lumens 60 in addition to, or instead of, being disposed in the central lumen 60.

Reference is now made to Fig. 17, which is an axial cross-sectional view of one of the cables 92 of Fig. 16. The strip electrodes 26 (Fig. 1) and the surface mountable electrodes 38 (Fig. 1) are electrically connected to the proximal end of the catheter 12 (Fig. 1) and the processor 44 (Fig. 1) and the ablation power generator 40 (Fig. 1), respectively, via insulated electrical wires 94 and insulated electrical wires 96. In other words, the strip electrodes 26 are electrically connected via the insulated electrical wires 94 and the surface mountable electrodes 38 are electrically connected via the insulated electrical wires 96. The insulated electrical wires 94 and the insulated electrical wires 96 are disposed in the cables 92 in the lumen(s) 60 (Fig. 16) of the deflectable element 16 (Fig. 16).

In some embodiments, the insulated electrical wires 94 and the insulated electrical wires 96 are disposed in the cables 92 using respective cable jackets 98 so that the cable jackets 98 are disposed in the deflectable element 16. In some embodiments, as shown in Fig. 17, several insulated electrical wires 94 are disposed in one of the cable jackets 98 with one of the insulated electrical wires 96. In general, respective groups of the insulated electrical wires 94 (e.g., grouped by the flexible polymer circuit strips 24 that the insulated electrical wires 94 are connected to) are disposed in respective ones of the cable jackets 98 (e.g., using an extruded tube of Perfluoroalkoxy alkane (PFA)), and respective ones of the insulated electrical wires 96 wires are disposed in respective ones of the cable jackets 98 among the respective groups of the insulated electrical wires 94. For example, the insulated electrical wires 94 and the insulated electrical wires 96 are grouped in the cable jackets 98 according to the flexible polymer circuit strips 24 to which the insulated electrical wires 94 and the insulated electrical wires 96 are electrically connected.

In some embodiments, the wire gauge of each of the insulated electrical wires 94 is greater than the wire gauge of each of the insulated electrical wires 96. The wire gauge of the insulated electrical wires 94 may have any suitable gauge, for example 48 AWG (American Wire Gauge), and may be formed from any suitable conductor, e.g., a copper-silver alloy, which provides extra strength for the thin wires 94. The wire gauge of the insulated electrical wires 96 may have any suitable gauge, for example 42 gauge, and may be formed from any suitable conductor, e.g., copper. The wire gauge of the insulated electrical wires 96 is typically chosen to support the currents supplied to the surface mountable electrodes 38 during IRE or RF ablation.

In some embodiments, the insulation of the insulated electrical wires 96 is thicker than the insulation of the insulated electrical wires 94. The thinner insulation of the insulated electrical wires 94 is generally more difficult to mechanically strip than the insulation of the insulated electrical wires 96.

The insulating material of the insulated electrical wires 94 may be formed from any suitable material, for example, but not limited to, high temperature polyurethane. The insulating material of the insulated electrical wires 96 may be formed from any suitable material, for example, but not limited to, high temperature polyimide.

In order to allow easy connection of the insulated electrical wires 94 to the contact arrays 64 (Fig. 9), the insulated electrical wires 94 include electrically insulating material 100 (only some labeled for the sake of simplicity) which is configured to have a temperature rating between 150 and 200 degrees Centigrade so that the electrically insulating material 100 melts or degrades (e.g., chars and crumbles) during soldering of the insulated electrical wires 94 to the contact arrays 64 (e.g., at a temperature of 300 degrees Centigrade) and therefore insulation of the insulated electrical wires 94 does not need to be mechanically stripped. The electrically insulating material 100 is more difficult to remove than the thicker insulation of the insulated electrical wires 96, when the insulation of the insulated electrical wires 96 is indeed thicker. In some embodiments, the insulated electrical wires 96 also include the electrically insulating material 100 which is configured to have a temperature rating between 150 and 200 so that the electrically insulating material 100 melts during soldering of the insulated electrical wires 96 to the contact arrays 64.

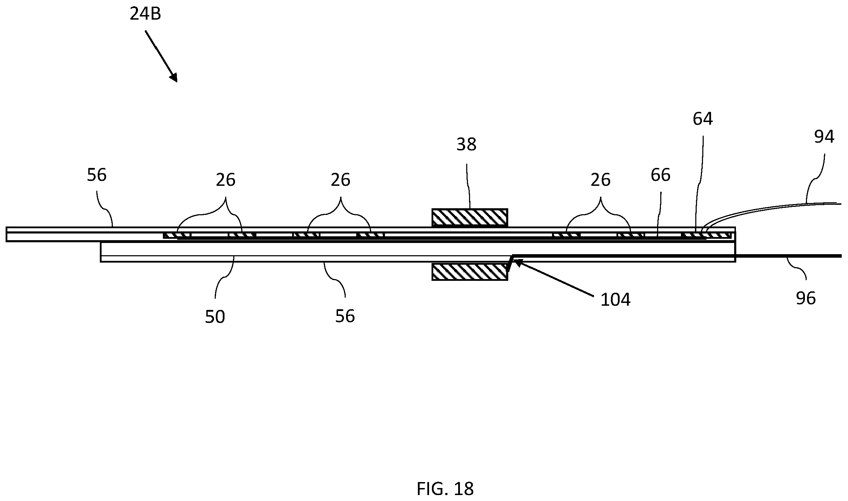

In accordance with embodiments of the invention, described in more detail with reference to Figs. 18-20, the insulated electrical wires 96 include electrically insulating material 102 which is configured to have a temperature rating greater than 200 degrees Centigrade to prevent the electrically insulating material 102 melting or degrading (e.g., charring and crumbling) during manufacture of the catheter 12 and/or during use. The electrically insulating material 102 is generally mechanically stripped before soldering the insulated electrical wires 96 to the surface mountable electrodes 38 as described in more detail below.

Reference is now made to Fig. 18, which is a schematic longitudinal cross-sectional view of an alternative flexible polymer circuit strip 24B for use with the catheter 12 of Fig. 2. **In** some embodiments, the flexible polymer circuit strips 24 described above with reference to Figs. 2-15 may be replaced with flexible polymer circuit strips 24B. The flexible polymer circuit strips 24B are substantially the same as the flexible polymer circuit strips 24 except for the differences described herein below.

Fig. 18 shows that the flexible polymer circuit strip 24B includes the multiple strip electrodes 26, the contact array 64 disposed at the proximal end of the flexible polymer circuit strip 24B, multiple circuit traces 66 (shown more clearly in Figs. 9 and 10) electrically connecting the multiple strip electrodes 26 with the contact array 64, and the elongated resilient support element 50. Therefore, when the flexible polymer circuit strips 24B replace the flexible polymer circuit strips 24, the catheter 12 (Fig. 1) includes respective flexible polymer circuit strips 24B including: respective multiple strip electrodes 26 (i.e., multiple strip electrodes 26 per flexible polymer circuit strip 24B); respective contact arrays 64 disposed at the respective proximal ends (i.e., one contact array 64 disposed at the proximal end of each flexible polymer circuit strip 24B); and respective multiple circuit traces 66 electrically connecting the respective multiple strip electrodes 26 with the respective contact arrays 64 (i.e., the circuit traces 66 of each flexible polymer circuit strip 24B electrically connect the contact array 64 to the strip electrodes 26 of that flexible polymer circuit strip 24B).

The catheter 12 also includes the surface mountable electrodes 38 mounted on and configured such that each electrode 38 can be seen to be bulging over respective ones of the flexible polymer circuit strips 24B. That is, each electrode 38 is disposed in such a way that the flexible circuit strip 24B is interposed into a hollow body portion such that the electrode 38 appears to be bulging or protruding from strip 24B such as seen in Figs. 7A and 11. For example, each of the flexible polymer circuit strips 24B may include one of the surface mountable electrodes 38 mounted thereon. As used herein, the terms "bulge", "bulging", "protrude", "protruding", "interpose" or "interposing" are used interchangeably to indicate the physical configuration of the larger electrode 38 disposed with respect to smaller strip 24B extending through larger electrode 38 along a central axis of electrode 38 such that larger electrode 38 appears to be bulging from strip 24B.

The respective groups of the insulated electrical wires 94 are electrically connected to the respective contact arrays 64 of respective ones of the flexible polymer circuit strips 24B. Fig. 18 shows one group of the insulated electrical wires 94 electrically connected to the flexible polymer circuit strip 24B of Fig. 18. Respective ones of the insulated electrical wires 96 are run externally to respective ones of the flexible polymer circuit strips 24B and are electrically connected to respective ones of the surface mountable electrodes 38. Fig. 18 shows one of the insulated electrical wires 96 running externally to the flexible polymer circuit strip 24B below the elongated resilient support element 50 and electrically connected to the surface mountable electrode 38.

The flexible polymer circuit strips 24B are covered with the coverings 56, e.g., respective shrink-sleeves formed from any suitable material such as PET shrink-tubing. The coverings 56 (e.g., shrink-sleeves) secure respective ones of the insulated electrical wires 96 to respective ones of the flexible polymer circuit strips 24B. Fig. 18 shows one of the insulated electrical wires 96 being secured to the flexible polymer circuit strip 24B via the elongated resilient support element 50. The covering 56 is typically shrunk using heat. Therefore, the electrically insulating material 102 (Fig. 17) of the insulated electrical wires insulated electrical wires 96 needs to be able to withstand the heat applied to the covering 56. If the electrically insulating material 102 melts or degrades, the conductor in the insulated electrical wire 96 may short with the elongated resilient support element 50, which may be formed from a metal such as Nitinol. Windows (not shown) are cut in the covering 56 above the strip electrodes 26 and the contact array 64. Fig. 18 shows the covering 56 prior to cutting the windows. For the sake of simplicity, Fig. 18 does not show the covering 56 at the ends of the flexible polymer circuit strip 24B and the elongated resilient support element 50.

The surface mountable electrode 38 of Fig. 18 is mounted externally to the covering 56 (e.g. shrink-sleeve) of the flexible polymer circuit strip 24B. In general, respective ones of the surface mountable electrodes 38 are mounted externally to the respective coverings 56 (e.g. shrink-sleeves) of respective ones of the flexible polymer circuit strips 24B. The surface mountable electrodes 38 may be affixed to the flexible polymer circuit strips 24B using a pressure fit and/or using a suitable adhesive and/or other securing method.

The insulated electrical wire 96 exits from the covering 56 via a hole 104 in the covering 56. The distal end of the insulated electrical wire 96 is manually stripped (e.g., using a suitable mechanical wire stripper) and is soldered to the surface mountable electrode 38.

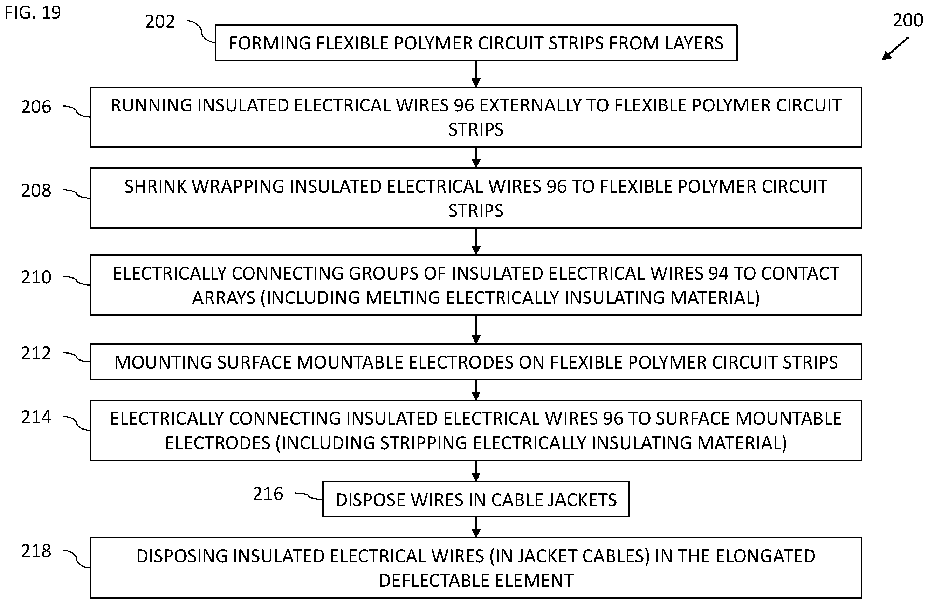

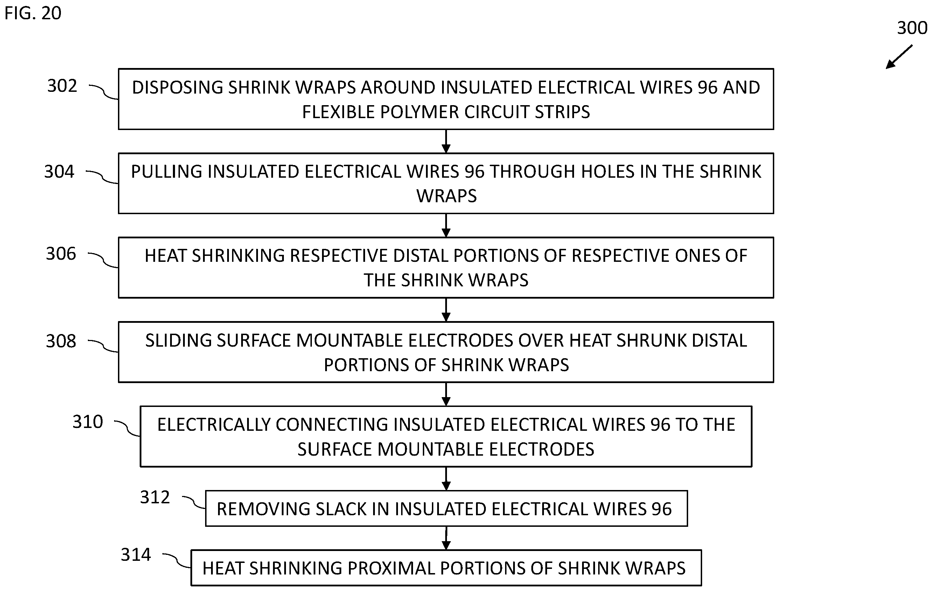

The manufacture of the cables 92 and the flexible polymer circuit strips 24B is described in more detail with reference to Figs. 19 and 20.

Reference is now made to Fig. 19, which is a flowchart 200 including steps in a method of manufacture of a catheter including the flexible polymer circuit strip 24B of Fig. 18. Reference is also made to Figs. 17 and 18.

The method includes forming or providing a catheter including: the deflectable element 16 (Fig. 1), the proximal coupler 20 (Fig. 1) connected to the distal end of the elongated deflectable element 16, and the expandable assembly 22 (Fig. 1) including the flexible polymer circuit strips 24B. The flexible polymer circuit strips 24B have respective proximal ends connected to, and disposed circumferentially around, the proximal coupler 20. Respective ones of the flexible polymer circuit strips 24B include: respective multiple strip electrodes 26; respective contact arrays 64 disposed at the respective proximal ends; and respective multiple circuit traces 66 electrically connecting the respective multiple strip electrodes 26 with the respective contact arrays 66. Formation of the flexible polymer circuit strips 24B is described in more detail below.

It should be noted that the steps listed below with reference to Figs. 19 and 20 may be performed in any suitable order and not just the order described below. The method includes forming (block 202) the flexible polymer circuit strips 24B from layers as described above with reference to Figs. 9 and 10. The method also includes running (block 206) respective ones of the insulated electrical wires 96 externally to respective flexible polymer circuit strips 24B and shrink-wrapping (block 208) respective ones of the insulated electrical wires 96 to respective ones of the flexible polymer circuit strips 24B. This step is generally performed in two stages as described in more detail with reference to Fig. 20. The method may also include cutting windows in the coverings 56 (e.g., shrink-sleeves) for the strip electrodes 26 and the contact arrays 64.

The method includes electrically connecting (block 210) (e.g., using soldering) respective groups of the insulated electrical wires 94 to the respective contact arrays 64 of respective ones of the flexible polymer circuit strips 24B. The electrically connecting of the step of block 210 also includes melting or degrading (e.g., from the heat of the soldering process) the electrically insulating material 100 of the insulated electrical wires 94 at the regions where the insulated electrical wires 94 join the contact arrays 64.

The method also includes mounting (block 212) the surface mountable electrodes 38 over respective ones of the flexible polymer circuit strips 24B with the surface mountable electrodes 38 bulging over the respective flexible polymer circuit strips 24B. The step of block 212 may also include sliding the surface mountable electrodes 38 over the coverings 56 (e.g., shrink-sleeves) of the respective flexible polymer circuit strips 24B.

The method also includes electrically connecting (block 214) (e.g., using soldering) respective ones of the insulated electrical wires 96 to respective ones of the surface mountable electrodes 38. The step of block 214 includes mechanically stripping the electrically insulating material 102 of the distal ends of the insulated electrical wires 96 prior to electrically bonding the insulated electrical wires 96 to the surface mountable electrodes 38.

The method also includes disposing (block 216) the respective groups of the insulated electrical wires 94 and respective ones of the insulated electrical wires 96 in respective ones of the cable jackets 98 of the respective cables 92. The step of block 216 may include disposing respective ones of the insulated electrical wires 96 in respective ones of the cable jackets 98 (i.e., one insulated electrical wire 96 per cable jacket 98) among the respective groups of the insulated electrical wires 94 (i.e., multiple insulated electrical wires 94 per cable jacket 98). In some embodiments, the insulated electrical wires 94 and the insulated electrical wires 96 are grouped according to their connections with the flexible polymer circuit strips 24B so that if the catheter 12 includes 8 flexible polymer circuit strips 24B, the catheter 12 also includes 8 corresponding cables 92 with cable jackets 98. The cable jackets 98 may be formed by extruding a tube (e.g., of PFA) over each wire bundle.

The method also includes disposing (block 218) the insulated electrical wires 94 and the insulated electrical wires 96 (which may be disposed in the cable jackets 98 of the cables 92) in the lumen(s) 60 (Fig. 16) of the deflectable element 16 (Fig. 16).

Reference is now made to Fig. 20, which is a flowchart 300 including more detailed steps in a method of manufacture of a catheter including the flexible polymer circuit strips 24B of Fig. 18.

The method includes disposing (block 302) respective coverings 56 (e.g., shrink-sleeves) around respective ones of the insulated electrical wires 96 and flexible polymer circuit strips 24B. The respective coverings 56 typically cover all sides of the respective flexible polymer circuit strips 24B including the respective elongated resilient support elements 50.

The method includes forming holes 104 in the coverings 56 (proximal to where the respective surface mountable electrodes 38 will be disposed on the respective flexible polymer circuit strips 24B), and pulling (block 304) respective distal ends of the insulated electrical wires 96 through the respective holes 104 in the respective coverings 56 (e.g., shrink-sleeves). The method also includes heat shrinking (block 306) respective distal portions (i.e., distal of the holes 104) of respective ones of the coverings 56 (e.g., shrink-sleeves).

The method includes sliding (block 308) respective ones of the surface mountable electrodes 38 over the heat shrunk distal portions of respective ones of the coverings 56 (e.g., shrink-sleeves) and electrically connecting (block 310) the distal ends of the respective insulated electrical wires 96 to respective ones of the surface mountable electrodes 38.

The method also includes removing slack (block 312) in respective ones of the insulated electrical wires 96, for example, by pulling the proximal ends of the insulated electrical wires 96. The method also includes heat shrinking (block 314) proximal portions (i.e., proximal of the holes 104) of respective ones of the coverings 56 (e.g., shrink-sleeves). The heat shrinking is performed in two stages as described above so that slack may be removed from the insulated electrical wires 96 after the insulated electrical wires 96 are connected to the surface mountable electrodes 38 which is after the surface mountable electrodes 38 are mounted on the distal end of the heat shrunk coverings 56.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 72% to 108%.

The embodiments described above are cited by way of example, and the present invention is not limited by what has been particularly shown and described hereinabove. Rather the scope of the invention is defined by the appended claims and includes both combinations and sub combinations of the various features described hereinabove, as well as variations and modifications thereof which fall within the scope of the claims and would occur to persons skilled in the art upon reading the foregoing description and which are not disclosed in the prior art.

## Claims

1. A medical system (10) including a catheter (12) configured to be inserted into a body part (14) of a living subject, and comprising:
an elongated deflectable element (16) including a distal end (18);
a proximal coupler (20) connected to the distal end;
an expandable assembly (22) comprising a plurality of flexible polymer circuit strips (24), the flexible polymer circuit strips having respective proximal ends connected to, and disposed circumferentially around, the proximal coupler, respective ones of the flexible polymer circuit strips including: respective multiple strip electrodes (26); respective contact arrays (64) disposed at the respective proximal ends; and respective multiple circuit traces electrically connecting the respective multiple strip electrodes with the respective contact arrays;
a plurality of surface mountable electrodes (38) mounted on, and bulging over respective ones of the flexible polymer circuit strips;
first insulated electrical wires (94) disposed in the elongated deflectable element, respective groups of the first insulated electrical wires being electrically connected to the respective contact arrays of respective ones of the flexible polymer circuit strips; and
second insulated electrical wires (96) disposed in the elongated deflectable element, respective ones of the second insulated electrical wires being run externally to respective ones of the flexible polymer circuit strips and being electrically connected to respective ones of the surface mountable electrodes,
wherein the first insulated electrical wires include electrically insulating material which is configured to have a temperature rating between 150 and 200 degrees Centigrade; and
the second insulated electrical wires include electrically insulating material which is configured to have a temperature rating greater than 200 degrees Centigrade.

2. The system according to claim 1, wherein the catheter includes respective shrink-sleeves (56) securing respective ones of the second wires (96) to respective ones of the flexible polymer circuit strips.

3. The system according to claim 2, wherein respective ones of the surface mountable electrodes are mounted externally to the respective shrink-sleeves of respective ones of the flexible polymer circuit strips.

4. The system according to claim **1,** wherein:
the catheter includes respective cable jackets (98) disposed in the elongated deflectable element;
the respective groups of the first insulated electrical wires (94) are disposed in respective ones of the cable jackets; and
respective ones of the second insulated electrical wires (96) are disposed in respective ones of the cable jackets among the respective groups of the first insulated wires.

5. The system according to any preceding claim, wherein each respective one of the surface mountable electrodes extends around the respective flexible polymer circuit strip.

6. The system according to any preceding claim, further comprising:
an ablation power generator (40) configured to be connected to the catheter, and apply an electrical signal to at least one of the surface mountable electrodes to ablate a tissue of the body part; and
a mapping module (46) configured to: receive electrical signals from ones of the strip electrodes of the flexible polymer circuit strips; and generate an electro-anatomical map (48) responsively to the received electrical signals.

7. The system according to any preceding claim, wherein:
the catheter includes a pusher (30) including a distal portion, and being configured to be advanced and retracted through the deflectable element;
the catheter includes a distal coupler (34) connected to the distal portion of the pusher; and
the flexible polymer circuit strips are disposed circumferentially around the distal portion of the pusher;
the flexible polymer circuit strips have respective distal ends connected to the distal coupler; and
the strips being configured to bow radially outward when the pusher is retracted expanding the expandable assembly from a collapsed form to an expanded form.

8. A catheter device configured to be inserted into a body part of a living subject, and comprising:
an elongated deflectable element including a distal end;
a distal end assembly disposed at the distal end and including a plurality of first electrodes and a plurality of second electrodes;
first insulated electrical wires disposed in the elongated deflectable element, respective ones of the first insulated electrical wires being electrically connected to respective ones of the first electrodes, the first insulated electrical wires including electrically insulating material which is configured to have a temperature rating between 150 and 200 degrees Centigrade; and
second insulated electrical wires disposed in the elongated deflectable element, respective ones of the second insulated electrical wires being electrically connected to respective ones of the second electrodes, the second insulated electrical wires including electrically insulating material which is configured to have a temperature rating greater than 200 degrees Centigrade.

9. The device according to claim 8, further comprising shrink-sleeves covering at least part of the second insulated electrical wires.

10. A method of manufacturing a catheter (12) comprising:
providing a catheter (12) including an elongated deflectable element (16), a proximal coupler (20) connected to a distal end (18) of the elongated deflectable element, and an expandable assembly (22) comprising a plurality of flexible polymer circuit strips (24), the flexible polymer circuit strips having respective proximal ends connected to, and disposed circumferentially around, the proximal coupler, respective ones of the flexible polymer circuit strips including: respective multiple strip electrodes (26); respective contact arrays (64) disposed at the respective proximal ends; and respective multiple circuit traces electrically connecting the respective multiple strip electrodes with the respective contact arrays;
mounting a plurality of surface mountable electrodes (38) over respective ones of the flexible polymer circuit strips with the surface mountable electrodes bulging over the respective ones of the flexible polymer circuit strips;
disposing first insulated electrical wires (94) in the elongated deflectable element, wherein the first insulated electrical wires include electrically insulating material which is configured to have a temperature rating between 150 and 200 degrees Centigrade;
electrically connecting respective groups of the first insulated electrical wires to the respective contact arrays of respective ones of the flexible polymer circuit strips, including melting electrically insulating material of the first wires;
disposing second insulated electrical wires (96) in the elongated deflectable element, wherein the second insulated electrical wires include electrically insulating material which is configured to have a temperature rating greater than 200 degrees Centigrade;
running respective ones of the second insulated electrical wires externally to respective one of the flexible polymer circuit strips; and
electrically connecting respective ones of the second insulated electrical wires to respective ones of the surface mountable electrodes, including mechanically stripping electrically insulating material of the second insulated electrical wires.

11. The system according to claim 1 or the method according to claim 10, wherein:
respective ones of the first insulated electrical wires (94) have a first wire gauge;
respective ones of the second insulated electrical wires (96) have a second wire gauge; and
the first wire gauge is greater than the second wire gauge.

12. The method according to claim 10 or claim **11,** further comprising shrink-wrapping respective ones of the second insulated electrical wires (96) to respective ones of the flexible polymer circuit strips.

13. The method according to claim 12, further comprising:
disposing respective shrink-sleeves (56) around respective ones of the second insulated electrical wires (96) and respective ones of the flexible polymer circuit strips;
pulling respective ones of the second insulated electrical wires through respective holes in respective ones of the shrink-sleeves;
heat shrinking respective distal portions of respective ones of the shrink-sleeves;
sliding respective ones of the surface mountable electrodes over the respective heat shrunk distal portions of respective ones of the shrink-sleeves;
electrically connecting respective ones of the second insulated electrical wires to respective ones of the surface mountable electrodes;
removing slack in respective ones of the second insulated electrical wires; and
heat shrinking respective proximal portions of respective ones of the shrink-sleeves.

14. The method according to claim 10, further comprising:
disposing the respective groups of the first insulated electrical wires (94) in respective cable jackets (98); and
disposing respective ones of the second insulated electrical wires (96) in respective ones of the cable jackets among the respective groups of the first insulated wires.

## Patentansprüche

1. Medizinisches System (10), das einen Katheter (12) einschließt, der konfiguriert ist, um in einen Körperteil (14) eines lebenden Subjekts eingeführt zu werden, und umfassend:
ein längliches, ablenkbares Element (16), das ein distales Ende (18) einschließt;
einen proximalen Koppler (20), die mit dem distalen Ende verbunden ist;
eine expandierbare Anordnung (22), umfassend eine Vielzahl von flexiblen Polymerschaltungsstreifen (24), wobei die flexiblen Polymerschaltungsstreifen jeweilige proximale Enden aufweisen, die mit dem proximalen Koppler verbunden und umlaufend um diesen herum angeordnet sind, wobei jeweilige der flexiblen Polymerschaltungsstreifen einschließen: jeweilige mehrere Streifenelektroden (26); jeweilige Kontaktarrays (64), die an den jeweiligen proximalen Enden angeordnet sind; und jeweilige mehrere Schaltungsbahnen, die die jeweiligen mehreren Streifenelektroden mit den jeweiligen Kontaktarrays elektrisch verbinden;
eine Vielzahl von auf der Oberfläche montierbaren Elektroden (38), die auf jeweiligen der flexiblen Polymerschaltungsstreifen montiert sind und sich über diese wölben;
erste isolierte elektrische Drähte (94), die in dem länglichen ablenkbaren Element angeordnet sind, wobei jeweilige Gruppen der ersten isolierten elektrischen Drähte mit den jeweiligen Kontaktarrays von jeweiligen der flexiblen Polymerschaltungsstreifen elektrisch verbunden sind; und
zweite isolierte elektrische Drähte (96), die in dem länglichen ablenkbaren Element angeordnet sind, wobei jeweilige der zweiten isolierten elektrischen Drähte außerhalb von jeweiligen der flexiblen Polymerschaltungsstreifen verlaufen und mit jeweiligen der auf der Oberfläche montierbaren Elektroden elektrisch verbunden sind,
wobei die ersten isolierten elektrischen Drähte elektrisch isolierendes Material einschließen, das konfiguriert ist, um eine Temperaturbeständigkeit zwischen 150 und 200 Grad Celsius aufzuweisen; und
die zweiten isolierten elektrischen Drähte elektrisch isolierendes Material einschließen, das konfiguriert ist, um eine Temperaturbeständigkeit von mehr als 200 Grad Celsius aufzuweisen.

2. System nach Anspruch 1, wobei der Katheter jeweilige Schrumpfschläuche (56) einschließt, die jeweilige der zweiten Drähte (96) an jeweiligen der flexiblen Polymerschaltungsstreifen befestigen.

3. System nach Anspruch 2, wobei jeweilige der auf der Oberfläche montierbaren Elektroden außen an den jeweiligen Schrumpfschläuchen von jeweiligen der flexiblen Polymerschaltungsstreifen montiert sind.

4. System nach Anspruch 1, wobei:
der Katheter jeweilige Kabelmäntel (98) einschließt, die in dem länglichen auslenkbaren Element angeordnet sind;
die jeweiligen Gruppen der ersten isolierten elektrischen Drähte (94) in jeweiligen der Kabelmäntel angeordnet sind; und
jeweilige der zweiten isolierten elektrischen Drähte (96) in jeweiligen der Kabelmäntel unter den jeweiligen Gruppen der ersten isolierten Drähte angeordnet sind.

5. System nach einem der vorstehenden Ansprüche, wobei sich jede jeweilige der auf der Oberfläche montierbaren Elektroden um den jeweiligen flexiblen Polymerschaltungsstreifen herum erstreckt.

6. System nach einem der vorstehenden Ansprüche, ferner umfassend:
einen Ablationsenergiegenerator (40), der konfiguriert ist, um mit dem Katheter verbunden zu werden und ein elektrisches Signal an mindestens eine der auf der Oberfläche montierbaren Elektroden anzulegen, um ein Gewebe des Körperteils zu abladieren; und
ein Kartierungsmodul (46), das konfiguriert ist zum: Empfangen elektrischer Signale von einigen der Streifenelektroden der flexiblen Polymerschaltungsstreifen; und Erzeugen einer elektroanatomischen Karte (48) als Reaktion auf die empfangenen elektrischen Signale.

7. System nach einem der vorstehenden Ansprüche, wobei:
der Katheter einen Schieber (30) einschließt, der einen distalen Abschnitt einschließt und konfiguriert ist, um durch das ablenkbare Element hindurch vorgeschoben und zurückgezogen zu werden;
der Katheter einen distalen Koppler (34) einschließt, der mit dem distalen Abschnitt des Schiebers verbunden ist; und
die flexiblen Polymerschaltungsstreifen umlaufend um den distalen Abschnitt des Schiebers herum angeordnet sind;
die flexiblen Polymerschaltungsstreifen jeweilige distale Enden aufweisen, die mit dem distalen Koppler verbunden sind; und
die Streifen konfiguriert sind, um sich radial nach außen biegen, wenn der Schieber zurückgezogen wird und die expandierbare Anordnung von einer zusammengeklappten Form zu einer expandierten Form expandiert.

8. Kathetervorrichtung, die konfiguriert ist, um in einen Körperteil eines lebenden Subjekts eingeführt zu werden, und umfassend:
ein längliches, ablenkbares Element, das ein distales Ende einschließt;
eine distale Endanordnung, die an dem distalen Ende angeordnet ist und eine Vielzahl von ersten Elektroden und eine Vielzahl von zweiten Elektroden einschließt;
erste isolierte elektrische Drähte, die in dem länglichen ablenkbaren Element angeordnet sind, wobei jeweilige der ersten isolierten elektrischen Drähte mit jeweiligen der ersten Elektroden elektrisch verbunden sind, wobei die ersten isolierten elektrischen Drähte elektrisch isolierendes Material einschließen, das konfiguriert ist, um eine Temperaturbeständigkeit zwischen 150 und 200 Grad Celsius aufzuweisen; und
zweite isolierte elektrische Drähte, die in dem länglichen ablenkbaren Element angeordnet sind, wobei jeweilige der zweiten isolierten elektrischen Drähte mit jeweiligen der zweiten Elektroden elektrisch verbunden sind, wobei die zweiten isolierten elektrischen Drähte elektrisch isolierendes Material einschließen, das konfiguriert ist, um eine Temperaturbeständigkeit von mehr als 200 Grad Celsius aufzuweisen.

9. Vorrichtung nach Anspruch 8, ferner umfassend Schrumpfschläuche, die mindestens einen Teil der zweiten isolierten elektrischen Drähte bedecken.

10. Verfahren zum Herstellen eines Katheters (12), umfassend:
Bereitstellen eines Katheters (12), der ein längliches ablenkbares Element (16), einen proximalen Koppler (20), der mit einem distalen Ende (18) des länglichen ablenkbaren Elements verbunden ist, und eine expandierbare Anordnung (22) einschließt, umfassend eine Vielzahl von flexiblen Polymerschaltungsstreifen (24), wobei die flexiblen Polymerschaltungsstreifen jeweilige proximale Enden aufweisen, die mit dem proximalen Koppler verbunden und umlaufend um diesen herum angeordnet sind, wobei jeweilige der flexiblen Polymerschaltungsstreifen einschließen: jeweilige mehrere Streifenelektroden (26); jeweilige Kontaktarrays (64), die an den jeweiligen proximalen Enden angeordnet sind; und jeweilige mehrere Schaltungsbahnen, die die jeweiligen mehreren Streifenelektroden mit den jeweiligen Kontaktarrays elektrisch verbinden;
Montieren einer Vielzahl von auf einer Oberfläche montierbaren Elektroden (38) über jeweiligen der flexiblen Polymerschaltungsstreifen, wobei sich die auf einer Oberfläche montierbaren Elektroden über die jeweiligen der flexiblen Polymerschaltungsstreifen wölben;
Anordnen erster isolierter elektrischer Drähte (94) in dem länglichen ablenkbaren Element, wobei die ersten isolierten elektrischen Drähte elektrisch isolierendes Material einschließen, das konfiguriert ist, um eine Temperaturbeständigkeit zwischen 150 und 200 Grad Celsius aufzuweisen;
elektrisches Verbinden jeweiliger Gruppen der ersten isolierten elektrischen Drähte mit den jeweiligen Kontaktarrays jeweiliger der flexiblen Polymerschaltungsstreifen, einschließlich eines Schmelzens von elektrisch isolierendem Material der ersten Drähte;
Anordnen zweiter isolierter elektrischer Drähte (96) in dem länglichen ablenkbaren Element, wobei die zweiten isolierten elektrischen Drähte elektrisch isolierendes Material einschließen, das konfiguriert ist, um eine Temperaturbeständigkeit von mehr als 200 Grad Celsius aufzuweisen;
Führen jeweiliger der zweiten isolierten elektrischen Drähte extern zu einem jeweiligen der flexiblen Polymerschaltungsstreifen; und
elektrisches Verbinden jeweiliger der zweiten isolierten elektrischen Drähte mit jeweiligen der auf der Oberfläche montierbaren Elektroden, einschließlich mechanischen Abisolierens von elektrisch isolierendem Material der zweiten isolierten elektrischen Drähte.

11. System nach Anspruch 1 oder Verfahren nach Anspruch 10, wobei:
jeweilige der ersten isolierten elektrischen Drähte (94) eine erste Drahtstärke aufweisen;
jeweilige der zweiten isolierten elektrischen Drähte (96) eine zweite Drahtstärke aufweisen; und
die erste Drahtstärke größer als die zweite Drahtstärke ist.

12. Verfahren nach Anspruch 10 oder 11, ferner umfassend ein Schrumpfverpacken jeweiliger der zweiten isolierten elektrischen Drähte (96) auf jeweilige der flexiblen Polymerschaltungsstreifen.

13. Verfahren nach Anspruch 12, ferner umfassend:
Anordnen jeweiliger Schrumpfschläuche (56) um jeweilige der zweiten isolierten elektrischen Drähte (96) und jeweilige der flexiblen Polymerschaltungsstreifen herum;
Ziehen jeweiliger der zweiten isolierten elektrischen Drähte durch jeweilige Löcher hindurch in jeweilige der Schrumpfschläuche;
Wärmeschrumpfen jeweiliger distaler Abschnitte jeweiliger der Schrumpfschläuche;
Aufschieben jeweiliger der auf der Oberfläche montierbaren Elektroden über die jeweiligen wärmegeschrumpften distalen Abschnitte von jeweiligen der Schrumpfschläuche;
elektrisches Verbinden jeweiliger der zweiten isolierten elektrischen Drähte mit jeweiligen der auf der Oberfläche montierbaren Elektroden;
Beseitigen durchhängender Teile in jeweiligen der zweiten isolierten elektrischen Drähte; und
Wärmeschrumpfen jeweiliger proximaler Abschnitte jeweiliger der Schrumpfschläuche.

14. Verfahren nach Anspruch 10, ferner umfassend:
Anordnen der jeweiligen Gruppen der ersten isolierten elektrischen Drähte (94) in jeweiligen Kabelmänteln (98); und
Anordnen jeweiliger der zweiten isolierten elektrischen Drähte (96) in jeweiligen der Kabelmäntel unter den jeweiligen Gruppen der ersten isolierten Drähte.

## Revendications

1. Système médical (10) comportant un cathéter (12) conçu pour être inséré dans une partie du corps (14) d'un sujet vivant, et comprenant :
un élément déformable allongé (16) comportant une extrémité distale (18) ;
un dispositif d'accouplement proximal (20) relié à l'extrémité distale ;
un ensemble expansible (22) comprenant une pluralité de bandes de circuit en polymère flexible (24), les bandes de circuit en polymère flexible ayant des extrémités proximales respectives reliées au dispositif d'accouplement proximal et disposées circonférentiellement autour de celui-ci, les bandes respectives des bandes de circuit en polymère flexible comportant : de multiples électrodes en bande respectives (26) ; des réseaux de contacts respectifs (64) disposés aux extrémités proximales respectives ; et de multiples pistes de circuit respectives connectant électriquement les multiples électrodes en bande respectives aux réseaux de contacts respectifs ;
une pluralité d'électrodes montables en surface (38) montées sur chacune des bandes de circuit en polymère flexible et faisant saillie au-dessus de celles-ci ;
des premiers fils électriques isolés (94) disposés dans l'élément déformable allongé, des groupes respectifs des premiers fils électriques isolés étant connectés électriquement aux réseaux de contacts respectifs de bandes de circuit en polymère flexible respectives ; et
des seconds fils électriques isolés (96) disposés dans l'élément déformable allongé, des fils respectifs des seconds fils électriques isolés étant acheminés à l'extérieur de bandes respectives des bandes de circuit en polymère flexible et étant connectés électriquement à des électrodes respectives des électrodes montables en surface,
dans lequel les premiers fils électriques isolés comportent un matériau isolant électriquement qui est configuré pour avoir une température nominale comprise entre 150 et 200 degrés centigrades ; et
les seconds fils électriques isolés comportent un matériau isolant électriquement qui est configuré pour avoir une température nominale supérieure à 200 degrés centigrades.

2. Système selon la revendication 1, dans lequel le cathéter comporte des manchons thermorétractables respectifs (56) fixant des seconds fils respectifs (96) à des bandes de circuit en polymère flexible respectives.

3. Système selon la revendication 2, dans lequel des électrodes montables en surface respectives sont montées à l'extérieur des manchons thermorétractables respectifs des bandes de circuit en polymère flexible respectives.

4. Système selon la revendication 1, dans lequel :
le cathéter comporte des gaines de câble respectives (98) disposées dans l'élément déformable allongé ;
les groupes respectifs des premiers fils électriques isolés (94) sont disposés dans des gaines de câble respectives ; et
des fils respectifs des seconds fils électriques isolés (96) sont disposés dans des gaines de câble respectives parmi les groupes respectifs des premiers fils isolés.

5. Système selon l'une quelconque revendication précédente, dans lequel chacune des électrodes montables en surface respectives s'étend autour de la bande de circuit en polymère flexible respective.

6. Système selon l'une quelconque revendication précédente, comprenant en outre :
un générateur d'énergie d'ablation (40) configuré pour être connecté au cathéter, et appliquer un signal électrique à au moins l'une des électrodes montables en surface pour ablater un tissu de la partie du corps ; et
un module de cartographie (46) configuré pour : recevoir des signaux électriques provenant des électrodes en bande des bandes de circuit en polymère flexible ; et générer une carte électro-anatomique (48) en réponse aux signaux électriques reçus.

7. Système selon l'une quelconque revendication précédente, dans lequel :
le cathéter comporte un poussoir (30) comportant une partie distale, et conçu pour être avancé et rétracté à travers l'élément flexible ;
le cathéter comporte un dispositif d'accouplement distal (34) relié à la partie distale du poussoir ; et
les bandes de circuit en polymère flexible sont disposées circonférentiellement autour de la partie distale du poussoir ;
les bandes de circuit en polymère flexible ont des extrémités distales respectives connectées au dispositif d'accouplement distal ; et
les bandes étant conçues pour s'incurver radialement vers l'extérieur lorsque le poussoir est rétracté, expansant l'ensemble expansible d'une forme rétractée à une forme expansée.

8. Dispositif de cathéter conçu pour être inséré dans une partie du corps d'un sujet vivant, et comprenant :
un élément déformable allongé comportant une extrémité distale ;
un ensemble d'extrémité distale disposé au niveau de l'extrémité distale et comportant une pluralité de premières électrodes et une pluralité de secondes électrodes ;
des premiers fils électriques isolés disposés dans l'élément déformable allongé, des fils respectifs des premiers fils électriques isolés étant connectés électriquement à des électrodes respectives des premières électrodes, les premiers fils électriques isolés comportant un matériau isolant électriquement qui est configuré pour avoir une température nominale comprise entre 150 et 200 degrés centigrades ; et
des seconds fils électriques isolés disposés dans l'élément déformable allongé, des fils respectifs des seconds fils électriques isolés étant connectés électriquement à des électrodes respectives des secondes électrodes, les seconds fils électriques isolés comportant un matériau isolant électriquement qui est configuré pour avoir une température nominale supérieure à 200 degrés centigrades.

9. Dispositif selon la revendication 8, comprenant en outre des manchons thermorétractables recouvrant au moins une partie des seconds fils électriques isolés.

10. Procédé de fabrication d'un cathéter (12), comprenant :
la fourniture d'un cathéter (12) comportant un élément déformable allongé (16), un dispositif d'accouplement proximal (20) relié à une extrémité distale (18) de l'élément déformable allongé, et un ensemble expansible (22) comprenant une pluralité de bandes de circuit en polymère flexible (24), les bandes de circuit en polymère flexible ayant des extrémités proximales respectives reliées au dispositif d'accouplement proximal et disposées circonférentiellement autour de celui-ci, des bandes respectives des bandes de circuit en polymère flexible comportant : de multiples électrodes en bande respectives (26) ; des réseaux de contacts respectifs (64) disposés aux extrémités proximales respectives ; et de multiples pistes de circuit respectives connectant électriquement les multiples électrodes en bande respectives aux réseaux de contacts respectifs ;
le montage d'une pluralité d'électrodes montables en surface (38) sur des bandes respectives des bandes de circuit en polymère flexible, les électrodes montables en surface faisant saillie sur les bandes respectives des bandes de circuit en polymère flexible ;
la disposition des premiers fils électriques isolés (94) dans l'élément déformable allongé, dans lequel les premiers fils électriques isolés comportent un matériau isolant électriquement qui est configuré pour avoir une température nominale comprise entre 150 et 200 degrés centigrades ;
la connexion de manière électrique des groupes respectifs des premiers fils électriques isolés aux réseaux de contacts respectifs de bandes de circuit en polymère flexible respectives, incluant la fusion du matériau isolant électriquement des premiers fils ;
la disposition des seconds fils électriques isolés (96) dans l'élément déformable allongé, dans lequel les seconds fils électriques isolés comportent un matériau isolant électriquement qui est configuré pour avoir une température nominale supérieure à 200 degrés centigrades ;
l'acheminement des fils respectifs des seconds fils électriques isolés à l'extérieur d'une bande respective des bandes de circuit en polymère flexible ; et
la connexion de manière électrique des fils respectifs des seconds fils électriques isolés à des électrodes respectives des électrodes montables en surface, incluant le dénudage de manière mécanique du matériau isolant électriquement des seconds fils électriques isolés.

11. Système selon la revendication 1 ou procédé selon la revendication 10, dans lequel :
des fils respectifs des premiers fils électriques isolés (94) ont un premier calibre de fil ;
des fils respectifs des seconds fils électriques isolés (96) ont un second calibre de fil ; et
le premier calibre de fil est supérieur au second calibre de fil.

12. Procédé selon la revendication 10 ou la revendication 11, comprenant en outre l'enveloppement thermorétractable des fils respectifs des seconds fils électriques isolés (96) sur des bandes respectives des bandes de circuit en polymère flexible.

13. Procédé selon la revendication 12, comprenant en outre :
la disposition des manchons thermorétractables respectifs (56) autour de fils respectifs des seconds fils électriques isolés (96) et de bandes respectives des bandes de circuit en polymère flexible ;
le tirage des seconds fils électriques isolés respectifs à travers des trous respectifs dans des manchons thermorétractables respectifs ;
la thermorétraction des parties distales respectives des manchons respectifs des manchons thermorétractables ;
le glissement des électrodes montables en surface respectives sur les parties distales thermorétractées respectives des manchons thermorétractables respectifs ;
la connexion de manière électrique des fils respectifs des seconds fils électriques isolés à des électrodes respectives des électrodes montables en surface ;
la suppression du mou dans les seconds fils électriques isolés respectifs ; et
la thermorétraction des parties proximales respectives des manchons respectifs des manchons thermorétractables.

14. Procédé selon la revendication 10, comprenant en outre :
la disposition des groupes respectifs des premiers fils électriques isolés (94) dans des gaines de câble respectives (98) ; et
la disposition des seconds fils électriques isolés (96) respectifs dans des gaines de câble respectives parmi les groupes respectifs des premiers fils isolés.
